# EUROPEAN PATENT APPLICATION

(11) **EP 3 866 140 A1**
(43) Date of publication of application: **18.08.2021**
(21) Application number: 21156386.1
(22) Date of filing: 10.02.2021
(51) Int. Cl.: G09B 9/00, G09B 23/28

(54) **SYSTEMS AND METHODS FOR SIMULATED PRODUCT TRAINING AND/OR EXPERIENCE**

(30) Priority: 11.02.2020 US 202062975106 P; 15.01.2021 US 202117150918
(71) Applicant: Covidien LP, Mansfield, MA 02048 (US)
(72) Inventor: GORDON, Brian C., Blaine, MN Minnesota 55449 (US); MOIN, Milad D., Eden Praire, MN Minnesota 55347 (US); SPERLING, Charles P., Edina, MN Minnesota 55436 (US)
(74) Representative: Maschio, Antonio

(57) **Abstract**

A virtual reality training system and method enabling virtual bronchoscopic navigation of a virtual patient following a pathway plan to a target. The pathway plan presented on a computer depicted in the virtual environment and simulating an actual bronchoscopic navigation.

## Description

### FIELD

Disclosed features concern medical training equipment and methods, and more particularly medical training equipment and methods used for training in bronchoscopic lung navigation procedures and techniques.

### BACKGROUND

There have been developed medical procedures on patients can involve a variety of different tasks by one or more medical personnel. Some medical procedures are minimally invasive surgical procedures performed using one or more devices, including a bronchoscope or an endoscope. In some such systems, a surgeon operates controls via a console, which remotely and precisely control surgical instruments that interact with the patient to perform surgery and other procedures. In some systems, various other components to the system can also be used to perform a procedure. For example, the surgical instruments can be provided on a separate instrument device or cart that is positioned near or over a patient, and a video output device and other equipment and devices can be provided on one or more additional units.

Systems have been developed to provide certain types of training in the use of such medical system. A simulator unit, for example, can be coupled to a surgeon console and be used in place of an actual patient, to provide a surgeon with a simulation of performing the procedure. With such a system, the surgeon can learn how instruments respond to manipulation and how those actions are presented or incorporated into the displays on the of the console.

However, these systems can be cumbersome to move and transport to various training sites. Further, because these systems are linked to actual consoles and other equipment there is both a capital cost for the training systems, and there is the potential need for maintenance on such systems. Accordingly, there is a need for improved training systems which address the shortcomings of these physical training aids

### SUMMARY

One aspect of the disclosure is directed to a training system for a medical procedure including: a virtual reality (VR) headset, including a processor and a computer readable recording media storing one or more applications thereon, the applications including instructions that when executed by the processor performs steps of: presenting a virtual environment viewable in the VR headset replicating a bronchoscopic suite including a patient, bronchoscopic tools, a patient, and a fluoroscope. The training system also includes depicting at least one representation of a user's hand in the virtual environment; providing instructions in the virtual environment viewable in the VR headset for performing a bronchoscopic navigation of the patient in the virtual environment; enabling interaction with a bronchoscopic navigation software on a computer displayed in the virtual environment; enabling interaction with the bronchoscopic tools via the representation of the user's hand; and executing a bronchoscopic navigation in the virtual environment, when the bronchoscopic navigation is undertaken, a user interface on the computer displayed in the virtual environment is updated to simulate a bronchoscopic navigation on an actual patient. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The training system further including a plurality of user interfaces for display on the computer in the virtual environment for performance of a local registration. The method further including a plurality of user interfaces for display on the computer in the virtual environment for performance of a local registration. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

One aspect of the disclosure is directed to a training system for a medical procedure including: a virtual reality (VR) headset; and a computer operably connected to the VR headset, the computer including a processor and a computer readable recording media storing one or more applications thereon, the applications including instructions that when executed by the processor performs steps of: presenting a virtual environment viewable in the VR headset replicating a bronchoscopic suite including a patient, bronchoscopic tools, a patient, and a fluoroscope; providing instructions in the virtual environment viewable in the VR headset for performing a bronchoscopic navigation of the patient in the virtual environment; enabling interaction with a bronchoscopic navigation software on a computer displayed in the virtual environment; and executing a bronchoscopic navigation in the virtual environment, when the bronchoscopic navigation is undertaken, a user interface on the computer displayed in the virtual environment is updated to simulate a bronchoscopic navigation on an actual patient. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The training system where the user interface displays one or more navigation plans for selection by a user of the VR headset. The training system where the computer in the virtual environment displays a user interface for performance of a registration of the navigation plan to a patient. The training system where during registration the virtual environment presents a bronchoscope, catheter, and locatable guide for manipulation by a user in the virtual environment to perform the registration. The training system where the virtual environment depicts at least one representation of a user's hands. The training system where the virtual environment depicts the user's hands manipulating the bronchoscope, catheter, or locatable guide. The training system where the virtual environment depicts the user's hands manipulating the user interface on the computer displayed in the virtual environment. The training system where the computer in the virtual environment displays a user interface for performance of navigation of airways of a patient. The training system where the user interface for performance of navigation includes central navigation, peripheral navigation, and target alignment. The training system where the user interface for performance of navigation depicts an updated position of the locatable guide as the bronchoscope or catheter are manipulated by a user. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

One aspect of the disclosure is directed to a method for simulating a medical procedure on a patient in a virtual reality environment, including: presenting in a virtual reality (VR) headset a virtual environment replicating a bronchoscopic suite including a patient, bronchoscopic tools, a patient, and a fluoroscope; providing instructions in the virtual environment viewable in the VR headset for performing a bronchoscopic navigation of the patient in the virtual environment; enabling interaction with a bronchoscopic navigation software on a computer displayed in the virtual environment; and executing a bronchoscopic navigation in the virtual environment, when the bronchoscopic navigation is undertaken, a user interface on the computer displayed in the virtual environment is updated to simulate a bronchoscopic navigation on an actual patient. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method where the virtual environment depicts at least one representation of a user's hands. The method where the virtual environment depicts the representation of the user's hands manipulating the bronchoscope, catheter, or locatable guide. The method where the virtual environment depicts the representation of the user's hands manipulating the user interface on the computer displayed in the virtual environment. The method where the computer in the virtual environment displays a user interface for performance of navigation of airways of a patient. The method where the user interface for performance of navigation includes central navigation, peripheral navigation, and target alignment. The method where the user interface for performance of navigation depicts an updated position of a catheter within the patient as the catheter is manipulated by a representation of the user's hands. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

### BRIEF DESCRIPTION OF THE DRAWINGS

Objects and features of the presently disclosed system and method will become apparent to those of ordinary skill in the art when descriptions of various embodiments thereof are read with reference to the accompanying drawings, of which:
FIG. 1 is a perspective view of a virtual reality system in accordance with the disclosure;
Fig. 2 is a perspective view of a system for navigating to a soft-tissue target via the airways network in accordance with the disclosure;
Fig. 3 is a view of a virtual reality environment in accordance with the disclosure;
Fig. 4 is a view of a virtual reality environment in accordance with the disclosure;
Fig. 5 is a view of a virtual reality environment in accordance with the disclosure;
Fig. 6 are flow chart representations of workflows enabled in the virtual reality environment in accordance with the disclosure;
Fig. 7 is a flow chart illustrating a method of navigation in accordance with an embodiment of the disclosure;
Fig. 8 is an illustration of a user interface presenting a view for performing registration in accordance with the present disclosure;
Fig. 9 is an illustration of the view of FIG. 8 with each indicator activated;
Fig. 10 is an illustration of a user interface, presenting a view for verifying registration in accordance with the present disclosure;
Fig. 11 is an illustration of a user interface presenting a view for performing navigation to a target further presenting a central navigation tab;
Fig. 12 is an illustration of the view of Fig. 11 further presenting a peripheral navigation tab;
Fig. 13 is an illustration of the view of Fig. 11 further presenting the peripheral navigation tab of FIG. 12 near the target;
Fig. 14 is an illustration of the view of Fig. 11 further presenting a target alignment tab;
Fig. 15 is an illustration of the user interface of the workstation of FIG. 2 presenting a view for marking a location of a biopsy or treatment of the target; and
Fig. 16 is an illustration of the user interface presenting a view for reviewing aspects of registration.
Fig. 17 is a flow chart of a method for identifying and marking a target in fluoroscopic 3D reconstruction in accordance with the disclosure;
Fig. 18 is a screen shot of a user interface for marking a target in a fluoroscopic image in accordance with the disclosure;
Fig. 19 is a screen shot of a user interface for marking a medical device target in a fluoroscopic image in accordance with the disclosure;
Fig. 20 is a flow chart of a method for confirming placement of a biopsy tool in a target in accordance with the disclosure;
Fig. 21 is a screen shot of a user interface for confirming placement of a biopsy tool in a target in accordance with the disclosure;
Fig. 22 is a view of the virtual reality environment of Figs. 3-5 depicting the bronchoscopic tools of a virtual procedure in accordance with the disclosure;
Fig. 23 is a view of the virtual reality environment providing guidance as to action the user should take to perform a procedure in accordance with the disclosure;
Fig. 24 is a view of the virtual reality environment providing guidance as to action the user should take to perform a procedure in accordance with the disclosure;
Fig. 25 is a view of the virtual reality environment providing guidance as to action the user should take to perform a procedure in accordance with the disclosure;
Fig. 26 is a view of the virtual reality environment providing guidance as to action the user should take to perform a procedure in accordance with the disclosure;
Fig. 27 is a view of the virtual reality environment enabling the user to interact with the navigation software as displayed on a virtual computer;
Fig. 28 is a view of the virtual reality environment providing guidance as to action the user should take to perform a procedure in accordance with the disclosure; and
Fig. 29 is a view of the virtual reality environment providing guidance as to action the user should take to perform a procedure in accordance with the disclosure.

### DETAILED DESCRIPTION

This disclosure is directed to a virtual reality (VR) system and method for training clinicians in the use of medical devices and the performance of one or more medical procedures. In particular, the disclosure is directed to systems and methods for performing VR bronchoscopic and endoscopic navigation, biopsy, and therapy procedures.

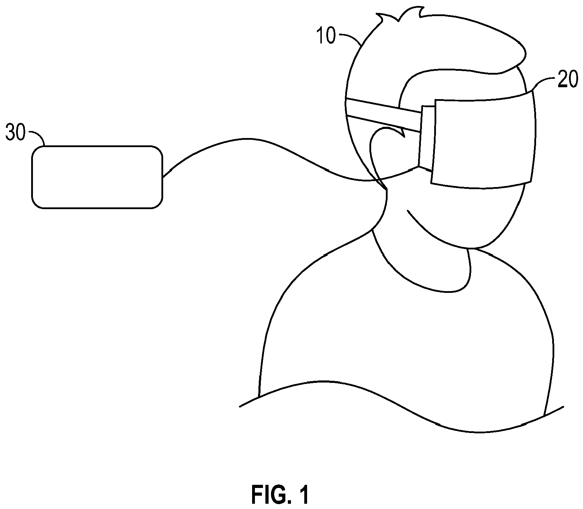

Fig. 1 depicts a clinician 10 wearing a virtual reality headset 20. The headset 20 may be for example a VR headset such as the Oculus Rift or Oculus Quest as currently sold by Facebook Technologies. The headset 20 may be connectable to a computer or may be a so called "all-in-one" system which is wirelessly connected to a phone or other access point to the internet. The headset 20 may be connected physically to a computer system 30 executing a variety of applications described herein.

The instant disclosure is directed to a VR bronchoscopic environment that allows for virtual reality access and operation of bronchoscopic navigation systems. There are several bronchoscopic navigation systems currently being offered including the Illumisite system offered by Medtronic PLC, the ION system offered by Intuitive Surgical Inc. the Monarch system offered by Auris, and the Spin system offered by Veran Medical Technologies. Though the disclosure focuses on implementation in the Illumisite system, the disclosure is not so limited and may be employed in any of these systems without departing from the scope of the disclosure.

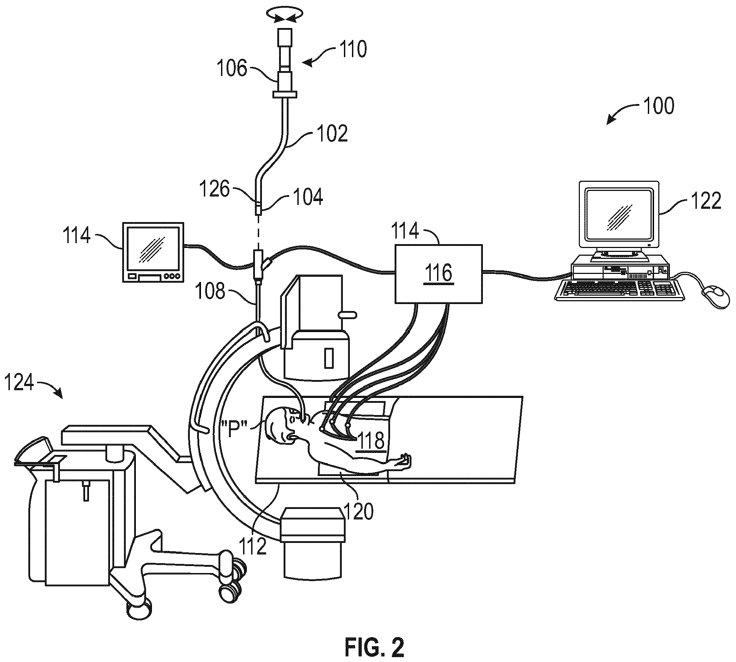

Fig. 2 generally depicts the Illumisite system 100 and the set-up for such a system in an operating room or bronchoscopy suite. As shown in Fig. 2, catheter 102 is part of a catheter guide assembly 106. In practice, catheter 102 is inserted into a bronchoscope 108 for access to a luminal network of the patient "P." Specifically, catheter 102 of catheter guide assembly 106 may be inserted into a working channel of bronchoscope 108 for navigation through a patient's luminal network. A locatable guide (LG) 110, including a sensor 104 is inserted into catheter 102 and locked into position such that sensor 104 extends a desired distance beyond the distal tip of catheter 102. The position and orientation of sensor 104 relative to the reference coordinate system, and thus the distal portion of catheter 102, within an electromagnetic field can be derived. Catheter guide assemblies 106 are currently marketed and sold by Medtronic PLC under the brand names SUPERDIMENSION® Procedure Kits, or EDGE™ Procedure Kits, and are contemplated as useable with the disclosure.

System 100 generally includes an operating table 112 configured to support a patient "P," a bronchoscope 108 configured for insertion through the patient "P"'s mouth into the patient "P"'s airways; monitoring equipment 114 coupled to bronchoscope 108 (e.g., a video display, for displaying the video images received from the video imaging system of bronchoscope 108); a locating or tracking system 114 including a locating or tracking module 116; a plurality of reference sensors 118; a transmitter mat 120 including a plurality of incorporated markers (not shown); and a computing device 122 including software and/or hardware used to facilitate identification of a target, pathway planning to the target, navigation of a medical device to the target, and/or confirmation and/or determination of placement of catheter 102, or a suitable device therethrough, relative to the target. Computing device 122 may be configured to execute the methods as described herein.

A fluoroscopic imaging device 124 capable of acquiring fluoroscopic or x-ray images or video of the patient "P" is also included in this particular aspect of system 100. The images, sequence of images, or video captured by fluoroscopic imaging device 124 may be stored within fluoroscopic imaging device 124 or transmitted to computing device 122 for storage, processing, and display. Additionally, fluoroscopic imaging device 124 may move relative to the patient "P" so that images may be acquired from different angles or perspectives relative to patient "P" to create a sequence of fluoroscopic images, such as a fluoroscopic video. The pose of fluoroscopic imaging device 124 relative to patient "P" while capturing the images may be estimated via markers incorporated with the transmitter mat 120. The markers are positioned under patient "P", between patient "P" and operating table 112, and between patient "P" and a radiation source or a sensing unit of fluoroscopic imaging device 124. The markers incorporated with the transmitter mat 120 may be two separate elements which may be coupled in a fixed manner or alternatively may be manufactured as a single unit. Fluoroscopic imaging device 124 may include a single imaging device or more than one imaging device.

Computing device 122 may be any suitable computing device including a processor and storage medium, wherein the processor is capable of executing instructions stored on the storage medium. Computing device 122 may further include a database configured to store patient data, CT data sets including CT images, fluoroscopic data sets including fluoroscopic images and video, fluoroscopic 3D reconstruction, navigation plans, and any other such data. Although not explicitly illustrated, computing device 122 may include inputs, or may otherwise be configured to receive CT data sets, fluoroscopic images/video, and other data described herein. Additionally, computing device 122 includes a display configured to display graphical user interfaces. Computing device 122 may be connected to one or more networks through which one or more databases may be accessed.

With respect to a planning phase, computing device 122 utilizes previously acquired CT image data for generating and viewing a three-dimensional model or rendering of the patient "P"'s airways, enables the identification of a target on the three-dimensional model (automatically, semi-automatically, or manually), and allows for determining a pathway through the patient "P"' s airways to tissue located at and around the target. More specifically, CT images acquired from previous CT scans are processed and assembled into a three-dimensional CT volume, which is then utilized to generate a three-dimensional model of the patient "P"' s airways. The three-dimensional model may be displayed on a display associated with computing device 122, or in any other suitable fashion. Using computing device 122, various views of the three-dimensional model or enhanced two-dimensional images generated from the three-dimensional model are presented. The enhanced two-dimensional images may possess some three-dimensional capabilities because they are generated from three-dimensional data. The three-dimensional model may be manipulated to facilitate identification of target on the three-dimensional model or two-dimensional images, and selection of a suitable pathway through the patient "P"' s airways to access tissue located at the target can be made. Once selected, the pathway plan, three-dimensional model, and images derived therefrom, can be saved, and exported to a navigation system for use during the navigation phase or phases.

With respect to the navigation phase, a six degrees-of-freedom electromagnetic locating or tracking system 114, or other suitable system for determining location or position, is utilized for performing registration of the images and the pathway for navigation, although other configurations are also contemplated. Tracking system 114 includes the tracking module 116, a plurality of reference sensors 118, and the transmitter mat 120 (including the markers). Tracking system 114 is configured for use with a locatable guide 110 and sensor 104. As described above, locatable guide 110 and sensor 104 are configured for insertion through catheter 102 into a patient "P"'s airways (either with or without bronchoscope 108) and are selectively lockable relative to one another via a locking mechanism.

Transmitter mat 120 is positioned beneath patient "P." Transmitter mat 120 generates an electromagnetic field around at least a portion of the patient "P" within which the position of a plurality of reference sensors 118 and the sensor 104 can be determined with use of a tracking module 116. A second electromagnetic sensor 126 may also be incorporated into the end of the catheter 102. Sensor 126 may be a five degree of freedom (5 DOF) sensor or a six degree of freedom (6 DOF) sensor. One or more of reference sensors 118 are attached to the chest of the patient "P." The six degrees of freedom coordinates of reference sensors 118 are sent to computing device 122 (which includes the appropriate software) where they are used to calculate a patient coordinate frame of reference. Registration is generally performed to coordinate locations of the three-dimensional model and two-dimensional images from the planning phase, with the patient "P"'s airways as observed through the bronchoscope 108 and allow for the navigation phase to be undertaken with precise knowledge of the location of the sensor 104, even in portions of the airway where the bronchoscope 108 cannot reach.

Registration of the patient "P"'s location on the transmitter mat 120 is performed by moving sensor 104 through the airways of the patient "P." More specifically, data pertaining to locations of sensor 104, while locatable guide 110 is moving through the airways, is recorded using transmitter mat 120, reference sensors 118, and tracking system 114. A shape resulting from this location data is compared to an interior geometry of passages of the three-dimensional model generated in the planning phase, and a location correlation between the shape and the three-dimensional model based on the comparison is determined, e.g., utilizing the software on computing device 122. In addition, the software identifies non-tissue space (e.g., air filled cavities) in the three-dimensional model. The software aligns, or registers, an image representing a location of sensor 104 with the three-dimensional model and/or two-dimensional images generated from the three-dimension model, which are based on the recorded location data and an assumption that locatable guide 110 remains located in non-tissue space in the patient "P"' s airways. Alternatively, a manual registration technique may be employed by navigating the bronchoscope 108 with the sensor 104 to pre-specified locations in the lungs of the patient "P", and manually correlating the images from the bronchoscope to the model data of the three-dimensional model.

Though described herein with respect to EMN systems using EM sensors, the instant disclosure is not so limited and may be used in conjunction with flexible sensor, ultrasonic sensors, or without sensors. Additionally, the methods described herein may be used in conjunction with robotic systems such that robotic actuators drive the catheter 102 or bronchoscope 108 proximate the target.

Following registration of the patient "P" to the image data and pathway plan, a user interface is displayed in the navigation software which sets for the pathway that the clinician is to follow to reach the target. Once catheter 102 has been successfully navigated proximate the target as depicted on the user interface, the locatable guide 110 may be unlocked from catheter 102 and removed, leaving catheter 102 in place as a guide channel for guiding medical devices including without limitation, optical systems, ultrasound probes, marker placement tools, biopsy tools, ablation tools (i.e., microwave ablation devices), laser probes, cryogenic probes, sensor probes, and aspirating needles to the target.

A medical device may be then inserted through catheter 102 and navigated to the target or to a specific area adjacent to the target. Upon achieving a position proximate the target, e.g., within about 2.5 cm, a sequence of fluoroscopic images may be then acquired via fluoroscopic imaging device 124 according to directions displayed via computing device 122. A fluoroscopic 3D reconstruction may be then generated via computing device 122. The generation of the fluoroscopic 3D reconstruction is based on the sequence of fluoroscopic images and the projections of structure of markers incorporated with transmitter mat 120 on the sequence of images. One or more slices of the 3D reconstruction may be then generated based on the pre-operative CT scan and via computing device 122. The one or more slices of the 3D reconstruction and the fluoroscopic 3D reconstruction may be then displayed to the user on a display via computing device 122, optionally simultaneously. The slices of 3D reconstruction may be presented on the user interface in a scrollable format where the user is able to scroll through the slices in series. The user may be then directed to identify and mark the target while using the slice of the 3D reconstruction as a reference. The user may be also directed to identify and mark the medical device in the sequence of fluoroscopic 2D-dimensional images. An offset between the location of the target and the medical device may be then determined or calculated via computing device 122. The offset may be then utilized, via computing device 122, to correct the location of the medical device on the display with respect to the target and/or correct the registration between the three-dimensional model and tracking system 114 in the area of the target and/or generate a local registration between the three-dimensional model and the fluoroscopic 3D reconstruction in the target area.

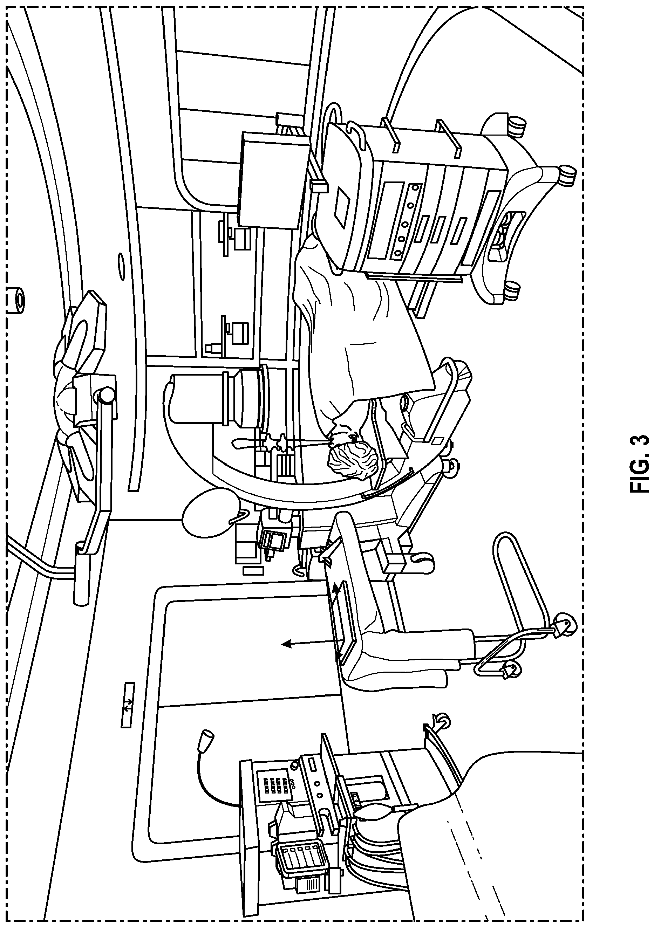

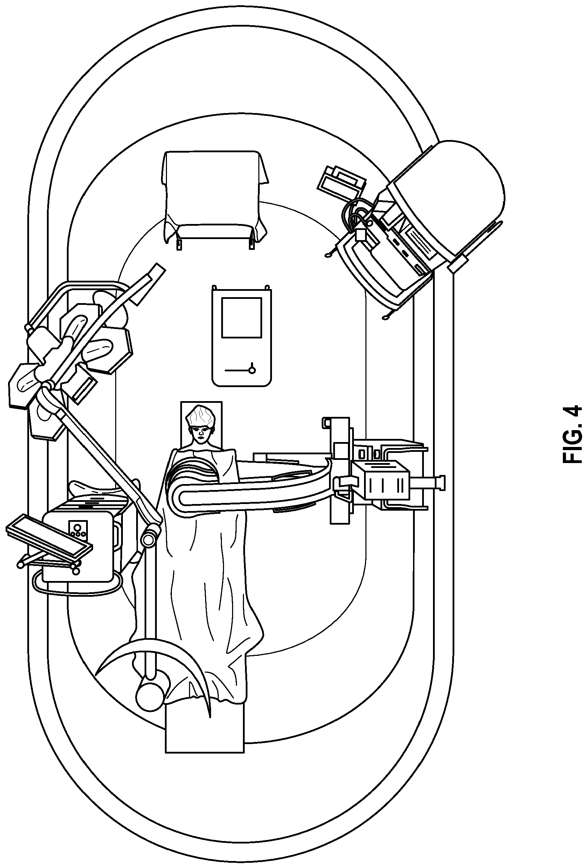

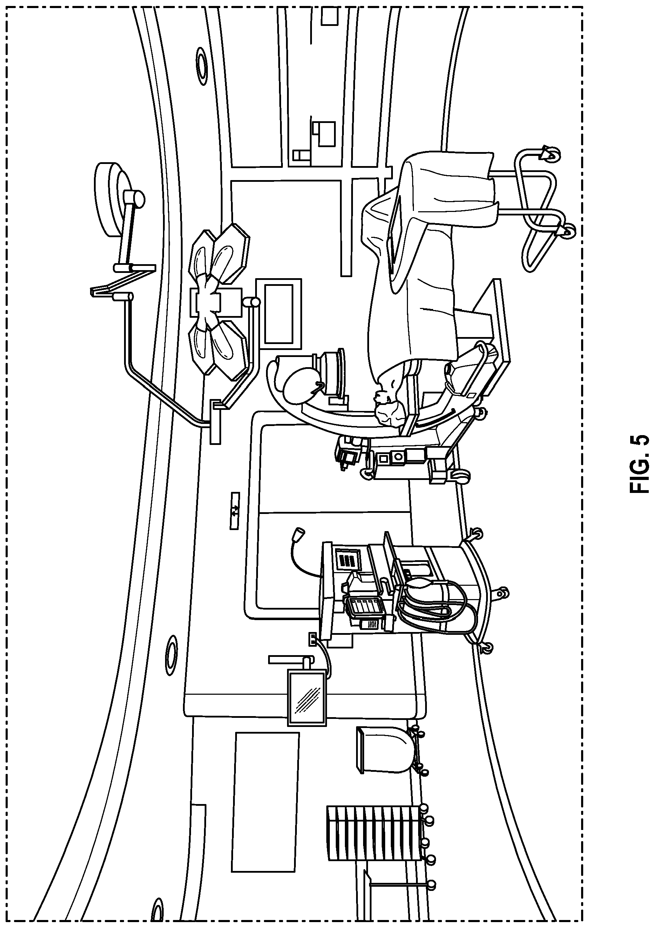

Fig. 3-5 depict a virtual environment viewable via the headset 20 to simulate the system 100 depicted in Fig. 2. Every component described above with respect to the actual system 100 are recreated in the virtual environments depicted in Figs 3-5. As will be described in greater detail below, these components in the virtual environment can be grasped, manipulated, and utilized in the virtual environment in connection with the headset 20. The headset 20 includes a variety of sensors which either alone or in combination with one or more handpieces (not shown) detect movement of the user's hands and head as the user moves through real space and translates these movements into the virtual reality as depicted to the user in the headset 20.

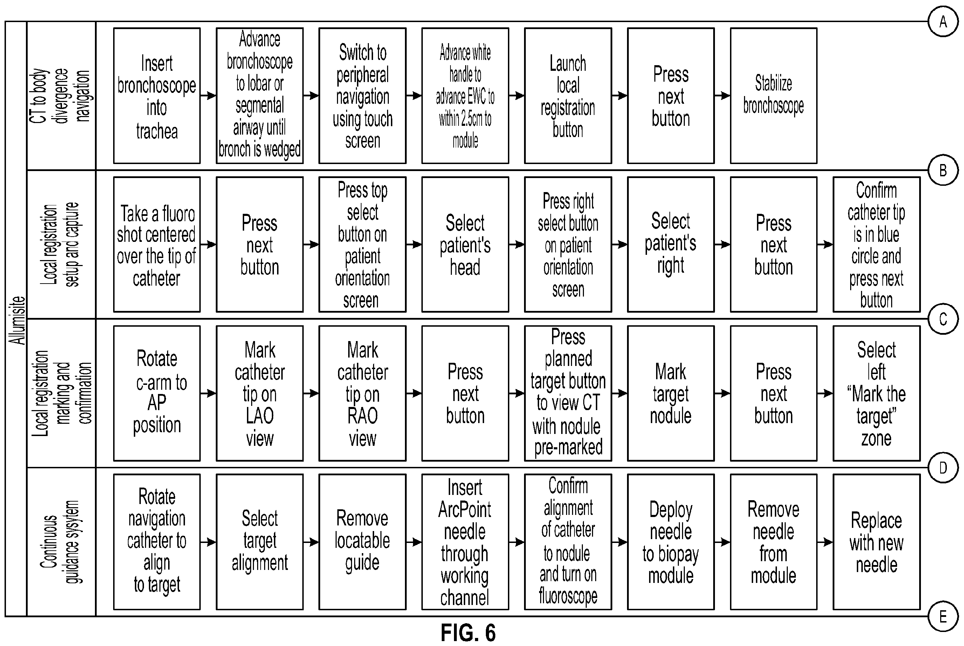

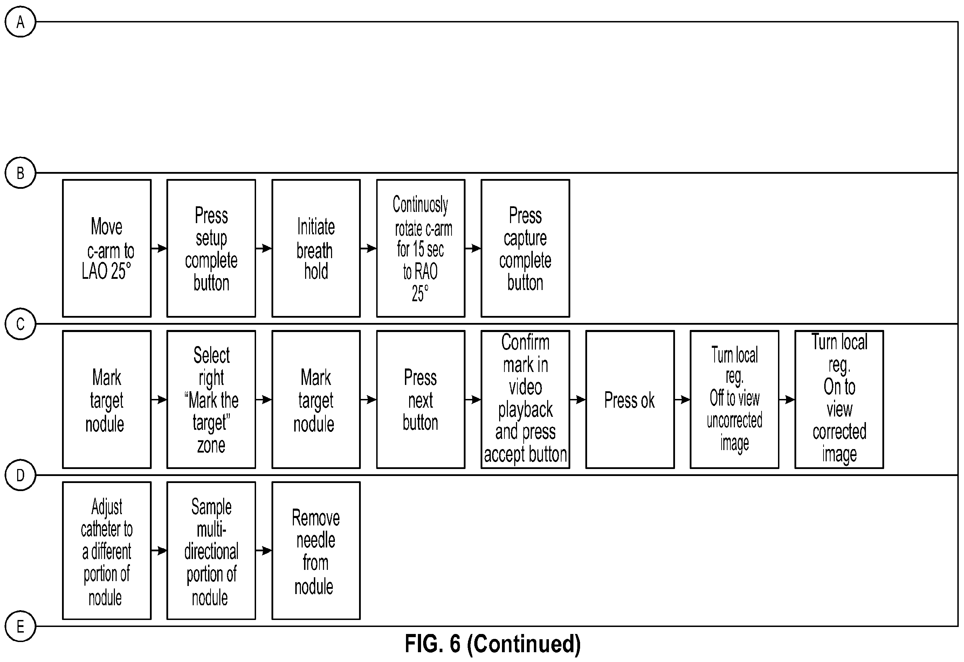

Fig. 6 depicts a variety of workflows for the system 100, which can be performed in the virtual environment utilizing the headset 20 and the computer 30. Each of these workflows is described in detail below or has been described in connection with the system 100, above. These workflows result in a series of displays in the headset 20 being presented to the user 10 as if they were undertaking a case and deploying an actual system 100. As a result, the user 10 can train without having to interact with an actual patient. Further, the computer 30 may collect data with which the performance of a user can be graded. In this way, a user can work in the virtual reality environment and undertake any number of procedures, learn the implementation details of the system 100 and be evaluated by experts without tying up equipment in the bronchoscopy suite, and without the need to engage an actual patient.

Further, the VR headset 20 and computer 30 can include an application that enables another user to be in the virtual environment. The second user may be another clinician that is demonstrating a case, a sales person, or an assistant who will be assisting the user in an actual case and needs to understand the functionality and uses of all the components of the system 100 prior to actual use.

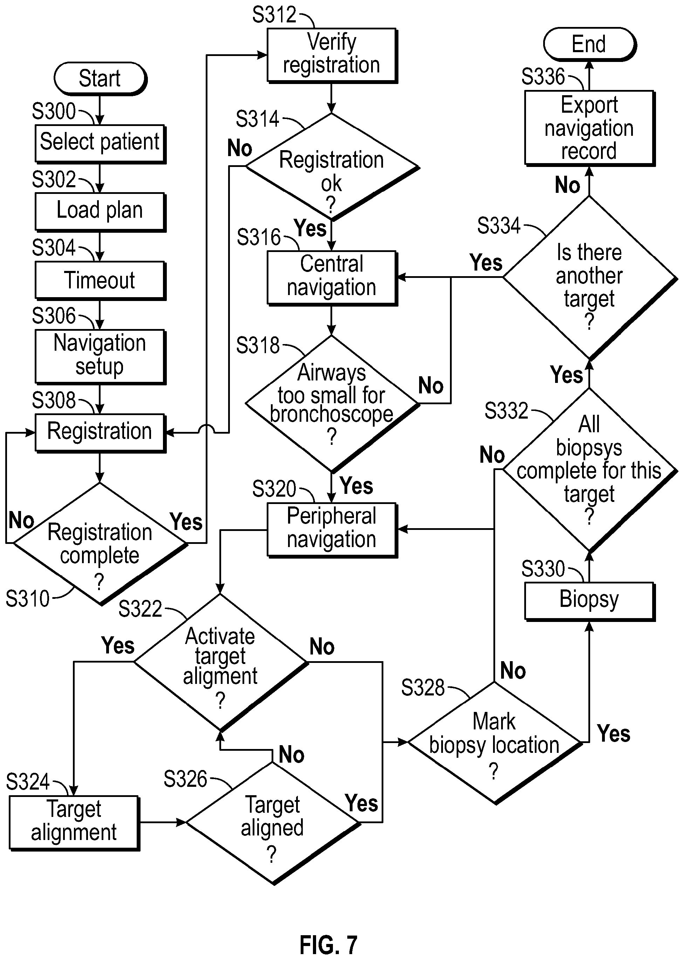

As noted above, the virtual environment enables a user wearing a headset 20 to perform virtual methods of navigation of a virtual representation of catheter 102 in a virtual patient. Fig. 7 describes these methods. In step S300, user interface, which is depicted on a virtual representation of the computer 122, presents the clinician with a view (not shown) for the selection of a patient. The clinician may enter patient information such as, for example, the patient name or patient ID number, into a text box to select a patient on which to perform a navigation procedure. Alternatively, the patient may be selected from a drop-down menu or other similar methods of patient selection. Once the patient has been selected, the user interface may present the clinician with a view (not shown) including a list of available navigation plans for the selected patient. In step S302, the clinician may load one of the navigation plans by activating the navigation plan. The navigation plans may be imported from a procedure planning software, described briefly above.

Once the patient has been selected and a corresponding navigation plan has been loaded, the user interface presents the clinician with a patient details view (not shown) in step S304 which allows the clinician to review the selected patient and plan details. Examples of patient details presented to the clinician in the timeout view may include the patient's name, patient ID number, and birth date. Examples of plan details include navigation plan details, automatic registration status, and/or manual registration status. For example, the clinician may activate the navigation plan details to review the navigation plan and may verify the availability of automatic registration and/or manual registration. The clinician may also activate an edit button (not shown) to edit the loaded navigation plan from the patient details view. Activating the edit button (not shown) of the loaded navigation plan may also activate the planning software described above. Once the clinician is satisfied that the patient and plan details are correct, the clinician proceeds to navigation setup in step S306. Alternatively, medical staff may perform the navigation setup prior to or concurrently with the clinician selecting the patient and navigation plan.

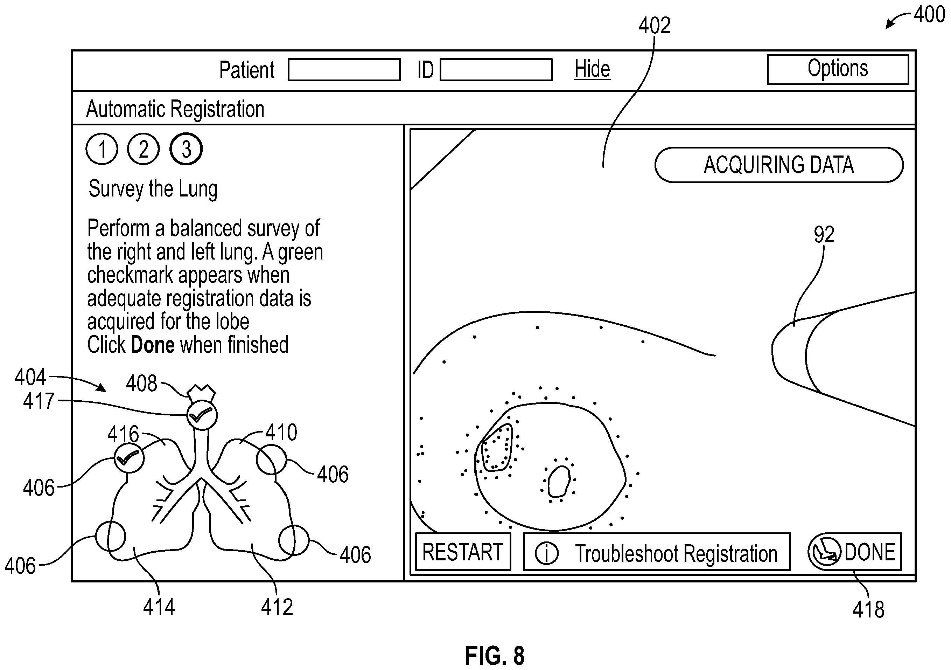

Once setup is complete, the user interface presents the clinician with a view 400 (FIG. 8) for registering the location of LG 110 relative to the loaded navigation plan. In step S308 the clinician prepares for registration by inserting bronchoscope 108 with catheter 102, LG 110 and EM sensor 104 into the virtual patient's airway until the distal ends of the LG 110, the EM sensor 104, and bronchoscope 108 are positioned within the patient's trachea, for example, as shown in FIG. 8. As shown in FIG. 8, view 400 presents a clinician with a video feed 402 from bronchoscope 108 and a lung survey 404. Video feed 402 from bronchoscope 108 provides the clinician with a real-time video of the interior of the patient's airways at the distal end of bronchoscope 108. Video feed 402 allows the clinician to visually navigate through the airways of the lungs.

Lung survey 404 provides the clinician with indicators 406 for the trachea 408 and each region 410, 412, 414, and 416 of the lungs. Regions 410, 412, 414, may also correspond to the patient's lung lobes. It is contemplated that an additional region (not shown) may be present and may correspond to the fifth lung lobe, e.g., the middle lung lobe in the patient's right lung. Lung survey 404 may also be modified for patients in which all or a part of one of the lungs is missing, for example, due to prior surgery.

During registration, the clinician advances bronchoscope 108 and LG 110 into each region 410, 412, 414, and 416 until the corresponding indicator 406 is activated. For example, the corresponding indicator may display a "check mark" symbol 417 when activated. As described above, the location of the EM sensor 104 of LG 110 relative to each region 410, 412, 414, and 416 is tracked by the electromagnetic interaction between EM sensor 104 of LG 110 and the electromagnetic field generator 120 and may activate an indicator 406 when the EM sensor 104 enters a corresponding region 410, 412, 414, or 416.

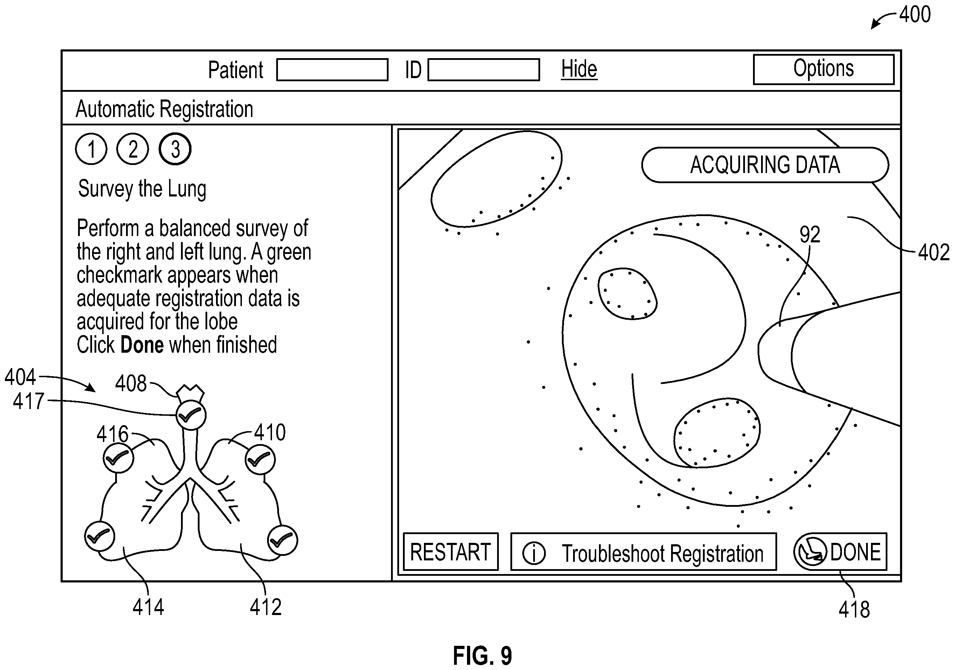

In step S310, once the indicators 406 for the trachea 408 and each region 410, 412, 414, and 416 have been activated, for example, as shown in FIG. 9, the clinician activates the "done" button 418 using, for example, a mouse or foot pedal, and proceeds to verification of the registration in step S312. Although each indicator 406 is shown as activated in FIG. 9, the clinician may alternatively achieve registration with the currently loaded navigation plan while one or more of regions 410, 412, 414, and 416 are not activated. For example, so long as the clinician has achieved sufficient registration with the currently loaded navigation plan the clinician may activate the "done" button 418 to proceed to registration verification in step S312. Sufficient registration may depend on both the patient's lung structure and the currently loaded navigation plan where, for example, only the indicators 406 for the trachea 408 and one or more of the regions 410, 412, 414, or 416 in one of the lungs may be necessary to achieve a useable registration where the plan identifies targets in one lung.

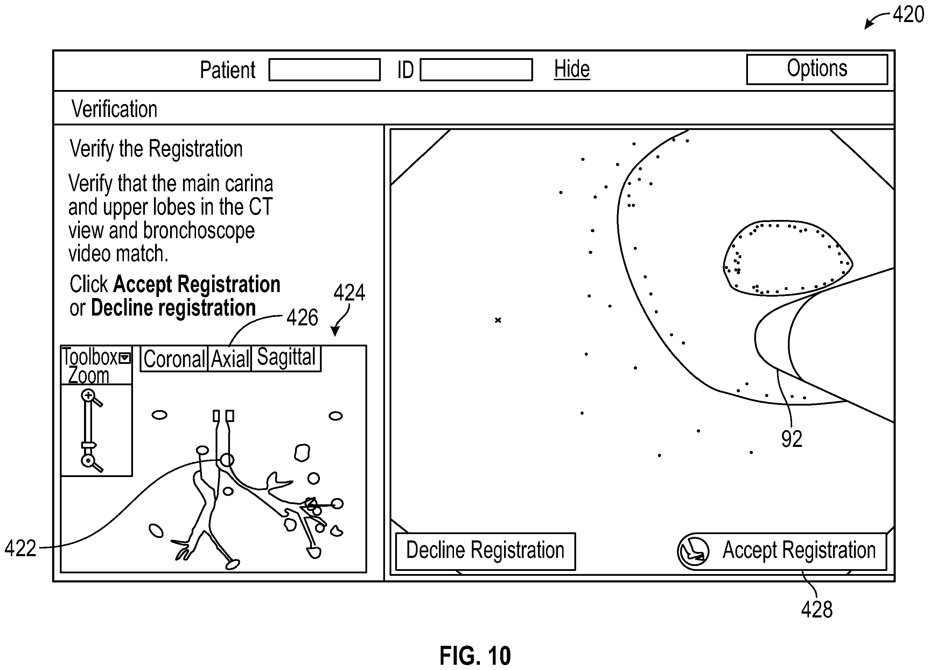

After registration with the navigation plan is complete, user interface presents the clinician with a view 420 for registration verification in step S312. View 420 presents the clinician with an LG indicator 422 (depicting the location of the EM sensor 104) overlaid on a displayed slice 424 of the CT images of the currently loaded navigation plan, for example, as shown in FIG. 10. Although the slice 424 displayed in FIG. 10 is from the coronal direction, the clinician may alternatively select one of the axial or sagittal directions by activating a display bar 426. As the clinician advances the LG 110 and bronchoscope 108 through the patient's airways, the displayed slice 424 changes based on the position of the EM sensor 104 of LG 110 relative to the registered 3D volume of the navigation plan. The clinician then determines whether the registration is acceptable in step S314. Once the clinician is satisfied that the registration is acceptable, for example, that the LG indicator 422 does not stray from within the patient's airways as presented in the displayed slice 424, the clinician accepts the registration by activating the "accept registration" button 428 and proceeds to navigation in step S316. Although registration has now been completed by the clinician, the EMN system 10 may continue to track the location of the EM sensor 104 of LG 110 within the patient's airways relative to the 3D volume and may continue to update and improve the registration during the navigation procedure.

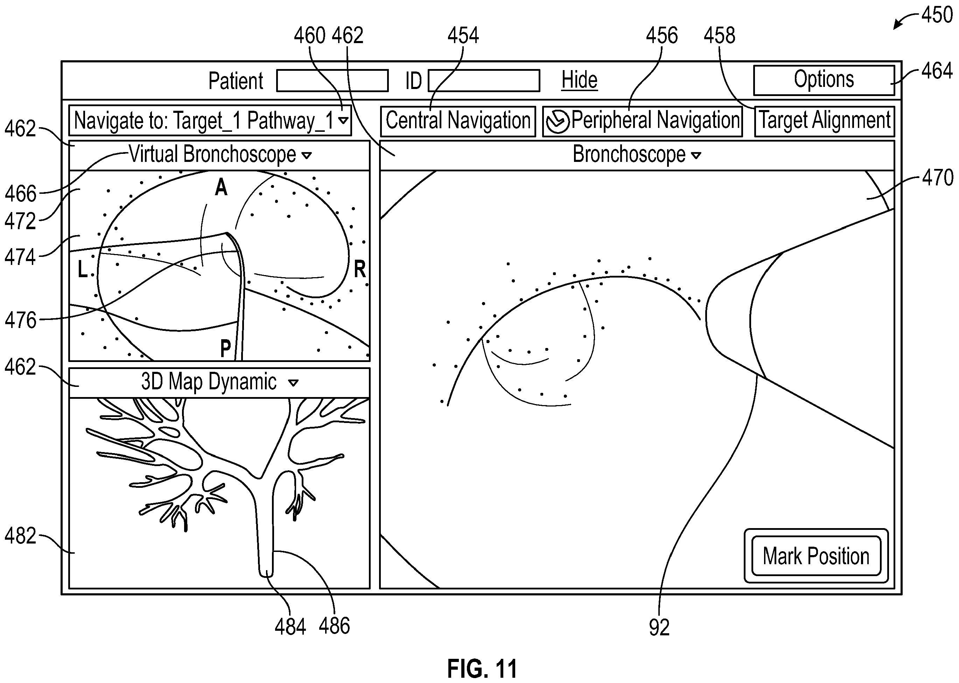

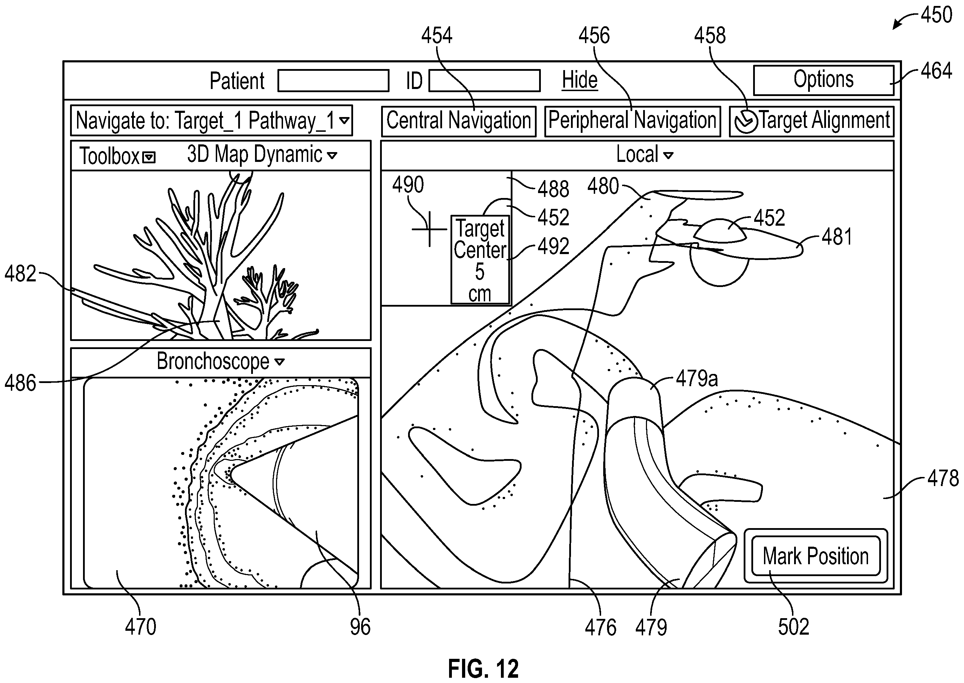

During navigation, a user interface on computer 122 presents the clinician with a view 450, as shown, for example, in FIG. 11. View 450 provides the clinician with a user interface for navigating to a target 452 (FIG. 12) including a central navigation tab 454, a peripheral navigation tab 456, and a target alignment tab 458. Central navigation tab 454 is primarily used to guide the bronchoscope 108 through the patient's bronchial tree until the airways become small enough that the bronchoscope 108 becomes wedged in place and is unable to advance. Peripheral navigation tab 456 is primarily used to guide the catheter 102, EM sensor 104, and LG 110 toward target 452 (FIG. 12) after the bronchoscope 108 is wedged in place. Target alignment tab 458 is primarily used to verify that LG 110 is aligned with the target 452 after LG 110 has been navigated to the target 452 using the peripheral navigation tab 456. View 450 also allows the clinician to select target 452 to navigate by activating a target selection button 460.

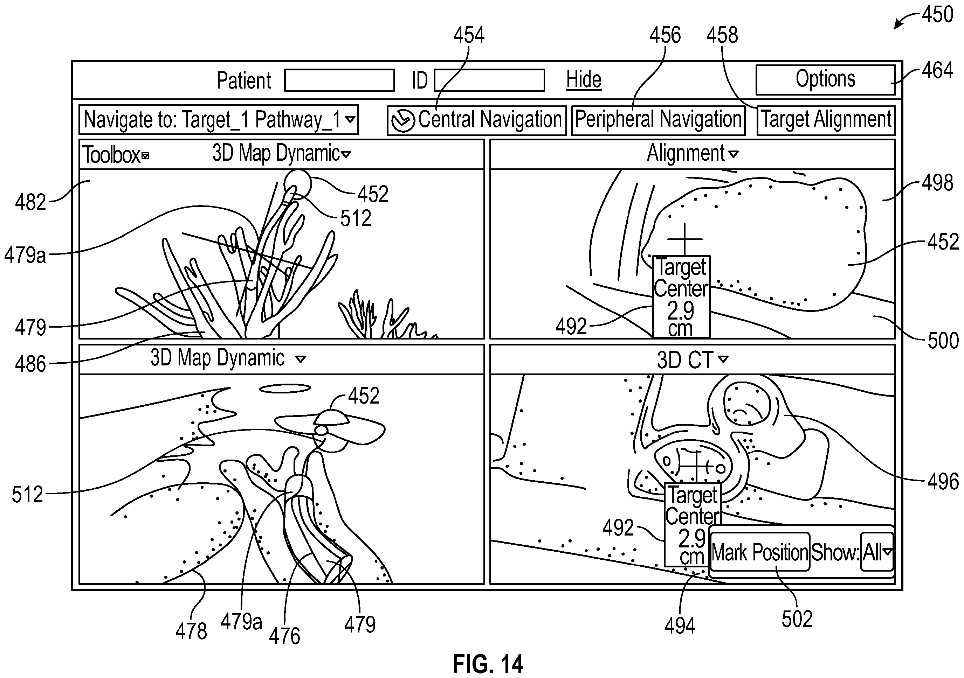

Each tab 454, 456, and 458 includes a number of windows 462 that assist the clinician in navigating to the target. The number and configuration of windows 462 to be presented is configurable by the clinician prior to or during navigation through the activation of an "options" button 464. The view displayed in each window 462 is also configurable by the clinician by activating a display button 466 of each window 462. For example, activating the display button 466 presents the clinician with a list of views for selection by the clinician including a bronchoscope view 470 (FIG. 11), virtual bronchoscope view 472 (FIG. 11), local view 478 (FIG. 12), MIP view (not explicitly shown), 3D map dynamic view 482 (FIG. 11), 3D map static view (not explicitly shown), sagittal CT view (not explicitly shown), axial CT view (not shown), coronal CT view (not explicitly shown), tip view 488 (FIG. 12), 3D CT view 494 (FIG. 14), and alignment view 498 (FIG. 14).

Bronchoscope view 470 presents the clinician with a real-time image received from the bronchoscope 108, as shown, for example, in FIG. 11. Bronchoscope view 470 allows the clinician to visually observe the patient's airways in real-time as bronchoscope 108 is navigated through the patient's airways toward target 452.

Virtual bronchoscope view 472 presents the clinician with a 3D rendering 474 of the walls of the patient's airways generated from the 3D volume of the loaded navigation plan, as shown, for example, in FIG. 11. Virtual bronchoscope view 472 also presents the clinician with a navigation pathway 476 providing an indication of the direction along which the clinician will need to travel to reach the target 452. The navigation pathway 476 may be presented in a color or shape that contrasts with the 3D rendering 474 so that the clinician may easily determine the desired path to travel.

Local view 478, shown in FIG. 12, presents the clinician with a slice 480 of the 3D volume located at and aligned with the distal tip of LG 110. Local view 478 shows target 452, navigation pathway 476, and surrounding airway branches overlaid on slice 480 from an elevated perspective. The slice 480 that is presented by local view 478 changes based on the location of EM sensor 104 relative to the 3D volume of the loaded navigation plan. Local view 478 also presents the clinician with a visualization of the distal tip 93 of LG 110 in the form of a virtual probe 479. Virtual probe 479 provides the clinician with an indication of the direction that distal tip 93 of LG 110 is facing so that the clinician can control the advancement of the LG 110 in the patient's airways. For example, as the clinician manipulates the handle of the catheter 102 and the LG 110 locked into position relative thereto rotate, and the orientation of the distal end 479 *a* of virtual probe 479 also rotates relative to the displayed slice 480 to allow the clinician to guide the LG 110 and catheter 102 through the patient's airways. The local view 478 also provides the clinician with a watermark 481 that indicates to the clinician the elevation of the target 452 relative to the displayed slice. For example, as seen in FIG. 12, the majority of the target 452 is located below watermark 481 and may, for example, be displayed as having a dark color such as a dark green, while a smaller portion of target 452 located above watermark 481 may be displayed, for example, as having a light color such as a light green. Any other color scheme which serves to indicate the difference between the portion of target 452 disposed above watermark 481 and the portion of target 452 disposed below watermark 481 may alternatively be used.

The MIP view (not explicitly shown), also known in the art as a Maximum Intensity Projection view is a volume rendering of the 3D volume of the loaded navigation plan. The MIP view presents a volume rendering that is based on the maximum intensity voxels found along parallel rays traced from the viewpoint to the plane of projection. For example, the MIP view enhances the 3D nature of lung nodules and other features of the lungs for easier visualization by the clinician.

3D map dynamic view 482 (FIG. 12) presents a dynamic 3D model 484 of the patient's airways generated from the 3D volume of the loaded navigation plan. Dynamic 3D model 484 includes a highlighted portion 486 indicating the airways along which the clinician will need to travel to reach target 452. The orientation of dynamic 3D model 484 automatically updates based on movement of the EM sensor 104 within the patient's airways to provide the clinician with a view of the dynamic 3D model 484 that is relatively unobstructed by airway branches that are not on the pathway to the target 452. 3D map dynamic view 482 also presents the virtual probe 479 to the clinician as described above where the virtual probe 479 rotates and moves through the airways presented in the dynamic 3D model 484 as the clinician advances the LG 110 through corresponding patient airways.

3D map static view (not explicitly shown) is similar to 3D map dynamic view 482 with the exception that the orientation of the static 3D model does not automatically update. Instead, the 3D map static view must be activated by the clinician to pan or rotate the static 3D model. The 3D map static view may also present the virtual probe 479 to the clinician as described above for 3D map dynamic view 482. The sagittal, axial, and coronal CT views (not explicitly shown) present slices taken from the 3D volume of the loaded navigation plan in each of the coronal, sagittal, and axial directions.

Tip view 488 presents the clinician with a simulated view from the distal tip 93 of LG 110, as shown, for example, in FIG. 12. Tip view 488 includes a crosshair 490 and a distance indicator 492. Crosshair 490 may be any shape, size, or color that indicates to the clinician the direction that the distal tip 93 of LG 110 is facing. Distance indicator 492 provides the clinician with an indication of the distance from the distal tip 93 of LG 110 to the center of target 452. Tip view 488 may be used to align the distal tip 93 LG 110 with the target 452.

3D CT view 494 (FIG. 14) presents the clinician with a 3D projection 496 of the 3D volume located directly in front of the distal tip of LG 110. For example, 3D projection 496 presents high density structures such as, for example, blood vessels, and lesions to the clinician. 3D CT view 494 may also present distance indicator 492 to the clinician as described above for tip view 488.

Alignment view 498 (FIG. 14) presents the clinician with a 2D projection 500 of the 3D volume located directly in front of the distal tip of LG 110, for example. 2D projection 500 presents high density structures such as, for example, blood vessels and lesions. In 2D projection 500, target 452 may presented as a color, for example, green, and may be translucent. Alignment view 498 may also present distance indicator 492 to the clinician as described above for tip view 488.

Navigation to a target 452 will now be described:

Initially, in step S316, view 450 is presented to the clinician by user interface with central navigation tab 454 active, as shown, for example, in FIG. 11. Central navigation tab 454 may be the default tab upon initialization of view 450 by user interface 202. Central navigation tab 454 presents the clinician with the bronchoscope view 470, virtual bronchoscope view 472, and 3D map dynamic view 482, as described above. Using central navigation tab 452, the clinician navigates bronchoscope 108, LG 110, and catheter 102 toward the target 452 by following the navigation pathway 476 of virtual bronchoscope view 472 along the patient's airways. The clinician observes the progress of bronchoscope 108 in each view 470, 472, and 482. In step S318, the clinician determines whether the airways leading to the target have become too small for bronchoscope 108 and, if so, wedges the bronchoscope 108 in place. Once the bronchoscope 108 has been wedged in place, the clinician activates peripheral navigation tab 456 , for example, a mouse or foot pedal, and proceeds to peripheral navigation in step S320.

During peripheral navigation in step S320, peripheral navigation tab 456 is presented to the clinician as shown, for example, in FIG. 12. Peripheral navigation tab 456 presents the clinician with the local view 478, 3D Map Dynamic view 482, bronchoscope view 470, and tip view 488. Peripheral navigation tab 456 assists the clinician with navigation between the distal end of bronchoscope 108 and target 452. As shown in the bronchoscope view 470 in FIG. 12, the clinician extends LG 110 and catheter 102 from the working channel of bronchoscope 108 into the patient's airway toward target 452. The clinician tracks the progress of LG 110, EM sensor 104, and catheter 102 in the local view 478, the 3D map dynamic view 482, and the tip view 488. For example, as described above, and shown in FIG. 12, the clinician rotates LG 110, EM sensor 104, and catheter 102 relative to the patient's airways until the tip 479 *a* of virtual probe 479 is oriented toward the desired airway leading to the target 452. For example, the desired airway may be determined based on the navigation pathway 476 presented in local view 478 and the highlighted portion 486 presented in 3D map dynamic view 482. The clinician then advances LG 110, EM sensor 104, and the catheter 102 into the desired airway and confirms the movement of the EM sensor 104 relative to the target 452 and the patient's airways in the 3D map dynamic view 482 and local view 478. The clinician may also check the location of target 452 on the tip view 488 to determine where the target 452 is relative to the orientation of the LG 110 as LG 110 moves closer to the target 452.

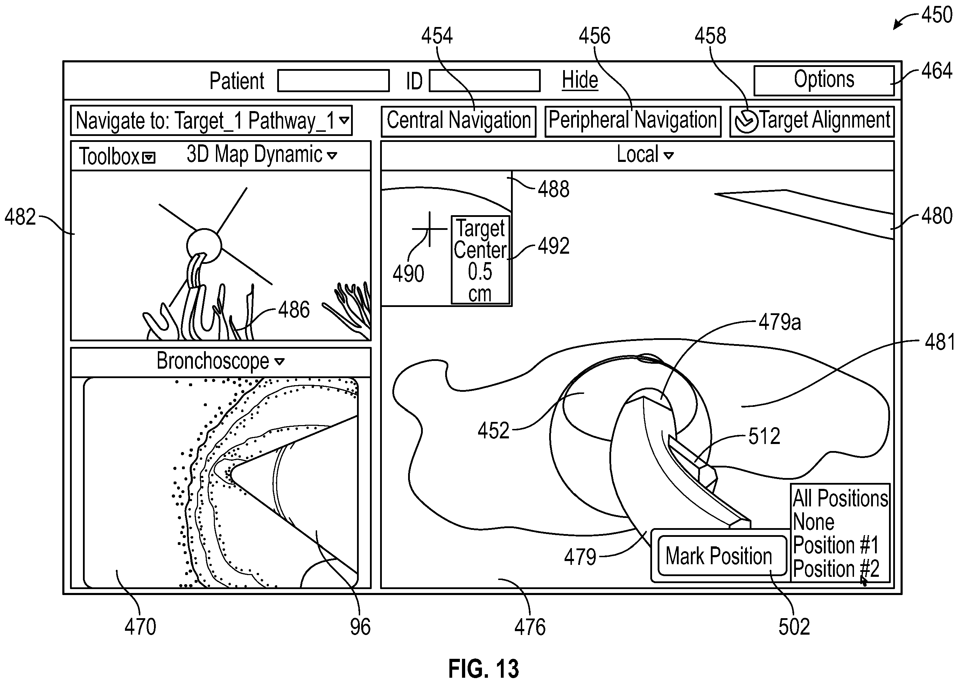

When the clinician has advanced the distal tip 93 of LG 110 to target 452, as shown, for example, in FIG. 13, the clinician may decide in step S322 to activate the target alignment tab 458 to confirm target alignment with the target 452.

During target alignment in step S324, target alignment tab 458 is presented to the clinician as shown, for example, in FIG. 14. Target alignment tab 458 presents the clinician with the local view 478, 3D Map Dynamic view 482, 3D CT view 494, and Alignment view 498. Target alignment tab 458 assists the clinician with alignment of the LG 110 with the target 452. By comparing the 3D and 2D projections of the 3D CT view 494 and alignment view 498 with the position and orientation of the virtual probe 479 in the local view 488 and 3D map dynamic view 482, the clinician may make a determination of whether the LG 110 is aligned with the target 452 and of the relative distance of the LG 110 to the target 452.

After the clinician determines that the target has been aligned in step S326 using the target alignment tab 458, or if the clinician decides not to activate the target alignment view 458 in step S322, the clinician may decide to activate the "mark position" button 502 of either the peripheral navigation tab 456 (FIG. 13) or the target alignment tab 458 (FIG. 14) in step S328 to virtually mark the current position of the virtual probe 479 where the registered position of the virtual probe 479 corresponds to the current location of the LG 110. This mark may be permanently recorded as part of the navigation plan to enable a clinician to return to substantially the same location in subsequent navigations or at a later time in the same procedure, for example, where a biopsy sample has been taken and is determined to be cancerous and in need of immediate treatment.

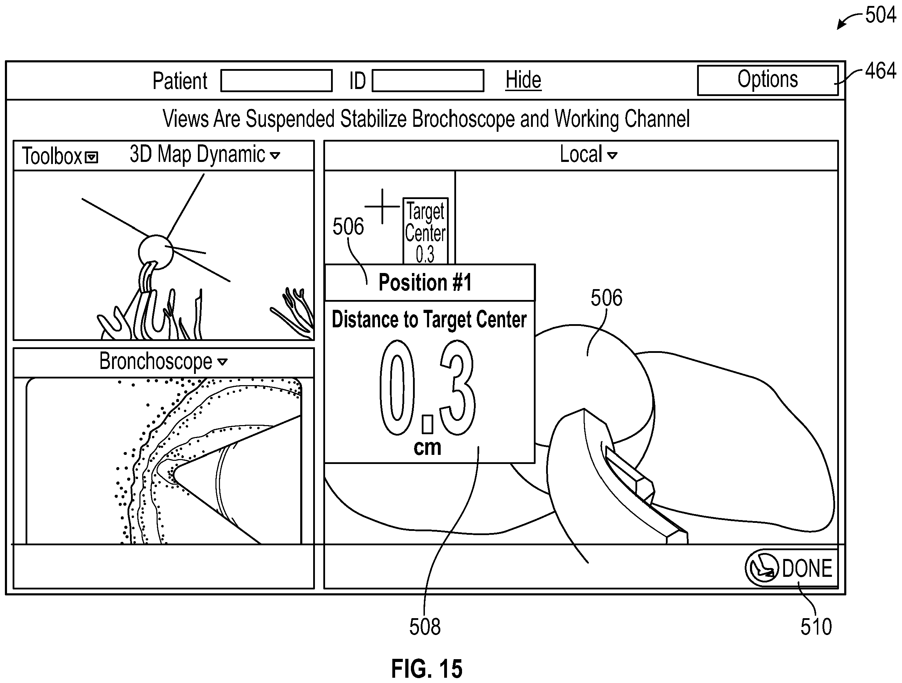

Once the clinician has activated the "mark position" button 502, the user interface presents the clinician with a view 504 providing the clinician with details of the marked position of the virtual probe 470, as shown, for example, in FIG. 15. For example, view 504 provides the clinician with a biopsy or treatment position number 506 and distance to target center 508 for the clinician's review. While view 504 is presented, the clinician may withdraw the LG 110 from catheter 102 of the bronchoscope 108 and insert a tool through catheter 102 in step S330, for example, a biopsy device 102, a fiducial marking device, an ablation probe, a chemical treatment probe, or other similar tools to sample, mark and/or treat the target 452. Once the clinician has finished sampling, marking, and/or treating the target 452 using the tool, the clinician withdraws the tool from bronchoscope 108 and inserts LG 110 back into bronchoscope 108. The clinician then activates the "done" button 510 to finish marking the target 452.

Once the "done" button 506 has been activated, the user interface presents the clinician with view 500 with one of tabs 454, 456, or 458 active. As can be seen in FIG. 14, for example, a representation of a virtual marker 512 is presented by target alignment tab 458 in various views including, for example, the 3D Map Dynamic view 482, local view 488, or any other view described above to indicate to the clinician the location of a previous treatment site. The clinician then determines whether an additional biopsy, marking, or treatment is required for the target 452 in step S332. If additional biopsies are required, the clinician repeats steps S320 through S330. Because the clinician has already navigated to the target 452, the clinician may alternatively repeat only a subset of steps S320 through S330. For example, the clinician may return to the target alignment tab 458 without activating the peripheral navigation tab 456 to continue navigating to the target or aligning the LG 110 with the target for an additional biopsy, marking, or treatment. Alternatively, the clinician may use only the peripheral navigation tab 456 to continue navigating to the target 452 for an additional biopsy or treatment.

If no additional biopsies or treatments are required, the clinician determines whether there is an additional target planned for navigation by activating the target selection button 460 in step S334. If an additional target is planned for navigation, the clinician activates the additional target and repeats steps S316 through S332 to navigate to the additional target for biopsy or treatment. If the additional target is in the same lung lobe or region as target 452, the clinician may alternatively only repeat a subset of steps S316 through S332. For example, the clinician may start navigation to the additional target using the peripheral navigation tab 456 (step S320) or the target alignment tab 458 (step S324) without using the central navigation tab 454 (step S316) where the location of the wedged bronchoscope 108 can still provide access to the additional target.

If there are no other targets, the clinician has finished the navigation procedure and may withdraw the LG 110, catheter 102, and bronchoscope 108 from the patient. The clinician may then export a record of the navigation procedure in step S336 to a memory associated with computer 122, or to a server or other destination for later review via network interface.

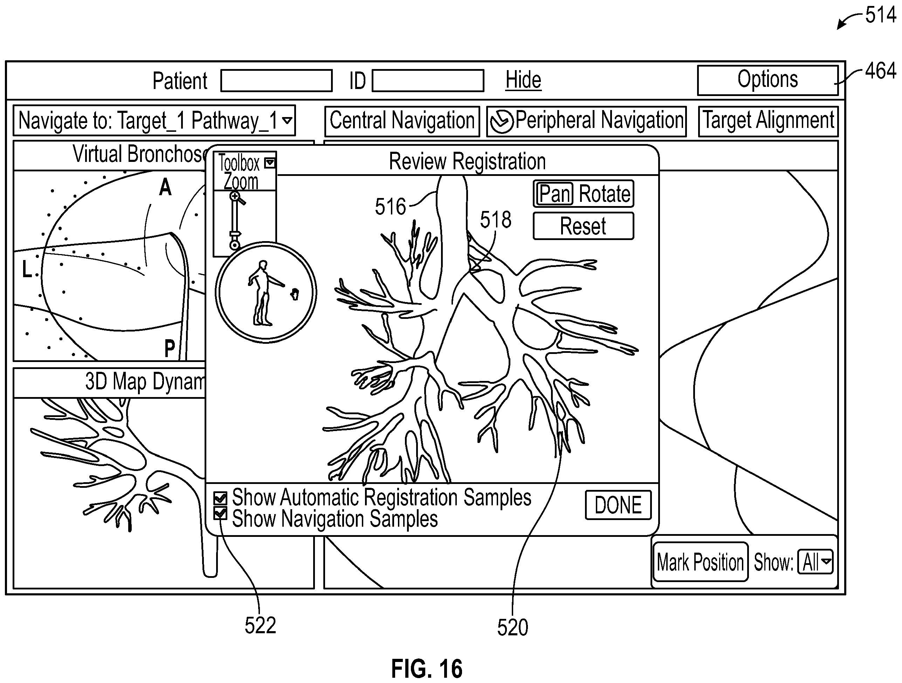

During the navigation procedure, the EM sensor 104 of LG 110 may continuously update registration information such that the registration is continuously updated. In addition, at any time during the navigation procedure the clinician may also review the registration by activating the "options" button 464 and activating a review registration button (not shown). The user interface then presents the clinician with a view 514 as shown, for example, in FIG. 16. View 514 presents the clinician with a 3D model 516 of the patient's bronchial tree generated from the 3D volume of the loaded navigation plan for review of the registration. As shown in FIG. 16, 3D model 516 includes a set of data points 518 that are generated during registration based on the locations to which the sensor 104 of LG 110 has traveled within the patient's airways. The data points 518 are presented on the 3D model 516 to allow the clinician to assess the overall registration of the 3D model 516 with the patient's airways. In addition, during navigation to target 452, a second set of data points 520 are generated based on the locations to which the sensor 104 of LG 110 has traveled on its path to the target 452. Data points 518 and 520 may be color coded, for example, green and purple, respectively, or may have different shapes or other identifying features that allow the clinician to differentiate between data points 518 and 520. The clinician may also activate or de-activate check boxes 522 to control which sets of data points 518 and 520 are presented in the 3D model 516.

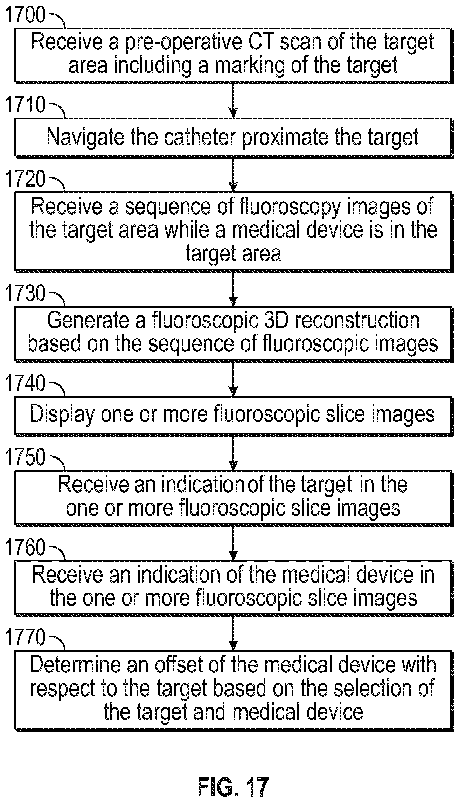

The above has been described using solely the navigation process without Fig. 17 depicts the process of performing a local registration using system 100. Where further confirmation of position is desired a local registration process may be undertaken in conjunction with the fluoroscopic imaging device 124. Utilizing the processes described above, a step 1700, a pre-operative CT scan of the target area may be received by the computing device 122. The pre-operative CT scan that is received may include a pathway plan that has already been developed or the user may generate the pathway plan using computing device 122. At step 1710 a user navigates the catheter 102 and sensor 104 proximate the target using the pathway plan which may be displayed on computing device 122 as part of the 3D model, described above.

Once proximate the target (e.g., about 2.5 cm), the user may wish to confirm the exact relative positioning of the sensor 104 and the target. At step 1720, a sequence of fluoroscopic images of the target area acquired in real time about a plurality of angles relative to the target area may be captured by fluoroscopic imaging device 124. The sequence of images may be captured while a medical device is positioned in the target area. In some embodiments, the method may include further steps for directing a user to acquire the sequence of fluoroscopic images. In some embodiments, the method may include one or more further steps for automatically acquiring the sequence of fluoroscopic images. The fluoroscopic images may be two-dimensional (2D) images, a three-dimensional (3D) reconstruction generated from a plurality of 2D images, or slice-images of a 3D reconstruction.

The disclosure refers to systems and methods for facilitating the navigation of a medical device to a target and/or a target area using two-dimensional fluoroscopic images of the target area. The navigation is facilitated by using local three-dimensional volumetric data, in which small soft-tissue objects are visible, constructed from a sequence of fluoroscopic images captured by a fluoroscopic imaging device. The fluoroscopic-based constructed local three-dimensional volumetric data may be used to correct a location of a medical device with respect to a target or may be locally registered with previously acquired volumetric data. In general, the location of the medical device may be determined by a tracking system such as the EM navigation system or another system as described herein. The tracking system may be registered with the previously acquired volumetric data.

In some embodiments, receiving a fluoroscopic 3D reconstruction of a body region may include receiving a sequence of fluoroscopic images of the body region and generating the fluoroscopic 3D reconstruction of the body region based on at least a portion of the fluoroscopic images. In some embodiments, the method may further include directing a user to acquire the sequence of fluoroscopic images by manually sweeping the fluoroscope. In some embodiments, the method may further include automatically acquiring the sequence of fluoroscopic images. The fluoroscopic images may be acquired by a standard fluoroscope, in a continuous manner and about a plurality of angles relative to the body region. The fluoroscope may be swept manually, i.e., by a user, or automatically. For example, the fluoroscope may be swept along an angle of 20 to 45 degrees. In some embodiments, the fluoroscope may be swept along an angle of 30±5 degrees. Typically, these images are gathered in a fluoroscopic sweep of the fluoroscopic imaging device 124 of about 30 degrees (i.e., 15 degrees on both sides of the AP position). As is readily understood, larger sweeps of 45, 60, 90 or even greater angles may alternatively be performed to acquire the fluoroscopic images.

At step 1730, a three-dimensional reconstruction of the target area may be generated based on the sequence of fluoroscopic images. In some embodiments, the method further comprises one or more steps for estimating the pose of the fluoroscopic imaging device while acquiring each of the fluoroscopic images, or at least a plurality of them. The three-dimensional reconstruction of the target area may be then generated based on the pose estimation of the fluoroscopic imaging device.

In some embodiments, the markers incorporated with the transmitter mat 120 may be placed with respect to the patient "P" and the fluoroscopic imaging device 124, such that each fluoroscopic image includes a projection of at least a portion of the structure of markers. The estimation of the pose of the fluoroscopic imaging device while acquiring each image may be then facilitated by the projections of the structure of markers on the fluoroscopic images. In some embodiments, the estimation may be based on detection of a possible and most probable projection of the structure of markers on each image.

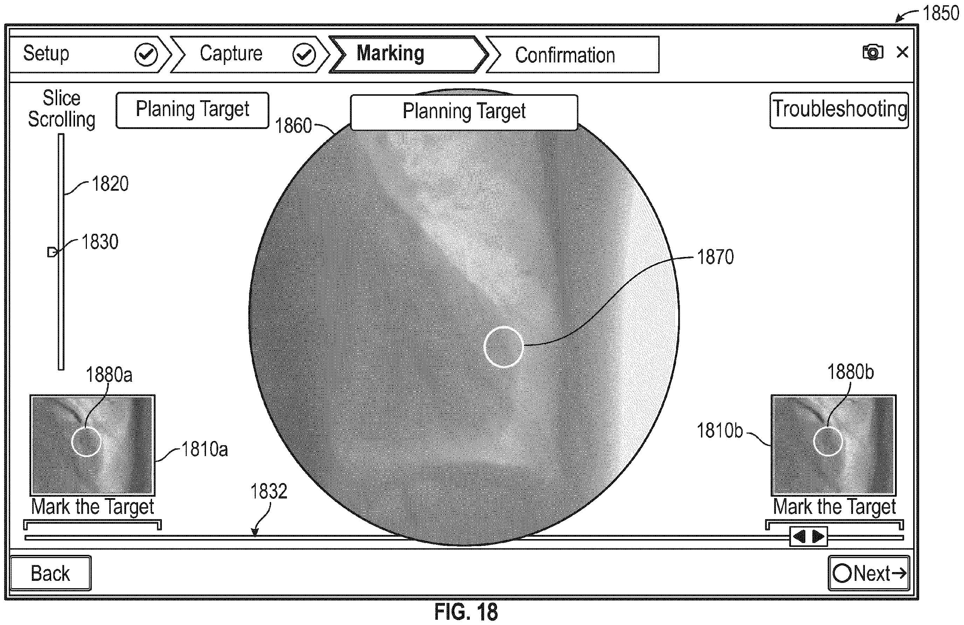

In step 1740, one or more fluoroscopy images are displayed to a user as illustrated in Fig. 18. As depicted in Fig. 18, which is an exemplary screen shot 1850 of a software program running on computing device 122, a slice of the 3D reconstruction 1860 is displayed simultaneously with two thumbnail images 1810a and 1810b of a fluoroscopic 3D reconstruction in accordance with the disclosure. As noted above, the 3D reconstruction is created for a fluoroscopic sweep of images between 30 and 90 degrees. By using the scroll bar 1820 and indicator 1830, the slice of the 3D reconstruction 1860 depicted on screen shot 1850 changes in accordance with the movement.

In some embodiments, when marking of the target in a slice image of a fluoroscopic 3D reconstruction is desired, generating and using a virtual slice image as a reference may be more advantageous. In some embodiments, when marking of the target in a fluoroscopic 2D image is desired, generating and using a virtual fluoroscopic 2D image may be more advantageous.

In accordance with step 1750, a selection of the target from the fluoroscopic 3D reconstruction is made by the user. As shown in Fig. 3A, at two end portions of the fluoroscopic 3D reconstruction displayable on screen shot 1850, the user is asked to "mark the target" in the slice of the 3D reconstruction 1860. These two ends could be any two positions of the fluoroscopic sweep, so long as they are sufficiently angularly separated such that efficient triangulation of the location of the target and the catheter (Fig. 18) can be performed.

As shown in Fig. 18, the user had previously marked the target at one end of the sweep, depicted in thumbnail image 180a, and is in the process of marking the target in a second end of the sweep, with the current slice of the 3D reconstruction 1860 which is depicted in thumbnail image 1810b. At the bottom of the screen shot 1850, two ranges are defined within which the user is to "mark the target" in an image that appears within a range. A second scroll bar 1832 on the bottom of the screen shot 350 allows the user to view the virtual fluoroscopy images 1860 as a video rather than to scroll through the slices individually using scroll bar 1820. In addition, the software may be configured to automatically jump the user from one "mark the target" range to the other following successful marking in the first "mark the target" range.

As depicted in Fig. 18, the user has placed a marker 1870 on the slice of the 3D reconstruction 1860. This marker also appears in thumbnail image 1810b as marker 1880b. In this manner, at step 1740 the user has marked the location of the target in the fluoroscopic image data collected by the fluoroscopic imaging device 124.

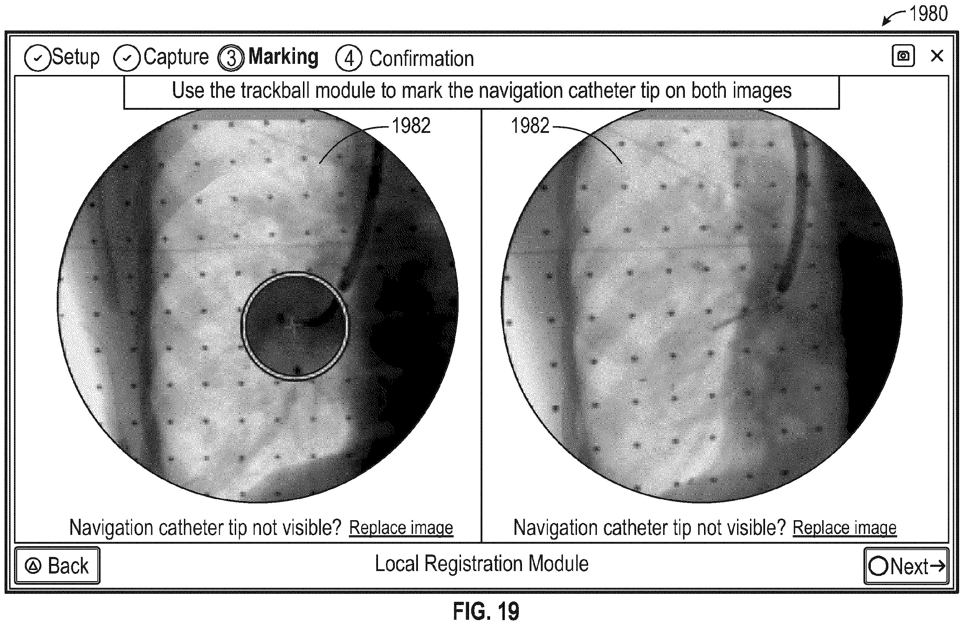

In step 1760, a selection of the medical device from the three-dimensional reconstruction or the sequence of fluoroscopic images is made. In some embodiments, this may be automatically made, and a user either accepts or rejects the selection. In some embodiments, the selection is made directly by the user. As depicted in Fig. 19, the user may be asked to mark the location of the catheter 102. Fig. 19 depicts a screen shot 1980 including two actual fluoroscopic images 1982. The user is asked to mark the end of the catheter 102 in each of the actual fluoroscopic images 1982. Each of these images comes from portions of the sweep that correspond to the "mark the target" portions depicted in Fig. 19.

Once both the catheter 102 and the target are marked at both ends of the sweep, at step 1770, an offset of the catheter 102 with respect to the target may be calculated. The determination of the offset is based on the received selections of the target and the medical device. This offset is used to update the detected position of the catheter 102, and specifically the sensor 104 in the 3D model and the pathway plan that was created to navigate to the target.

Typically, at this point in the procedure, the user has managed to navigate the catheter 102 to within 2-3 cm of the target, for example. With the updated position provided by the fluoroscopic data collection and position determination, the user can have confidence of reaching the target while traversing this last distance.

In heretofore known systems, the sensor 104 would now be removed from the catheter 102 and the final approaches to the target in navigation would proceed completely blind. Recently, systems have been devised that allow for the incorporation of a sensor 126 that can provide 5 DOF location information of the catheter 102 after the sensor 104 is removed.

Following removal of the sensor 104, a tool, such as a needle or coring biopsy tool, brushes, ablation devices (e.g., RF, microwave, chemical, radiological, etc.), clamping devices, and others, may be advanced down the catheter 102. In one example, a biopsy tool (not shown) is advanced down the catheter 102 and, using the sensor 126, the user navigates the final 2-3 cm, for example, to the target and can advance to biopsy tool into the target as it appears in the 3D model. However, despite the confidence provided by updating relative locations of the target and the catheter 102, there are times where a user may wish to confirm that the biopsy tool is in fact placed within the target.

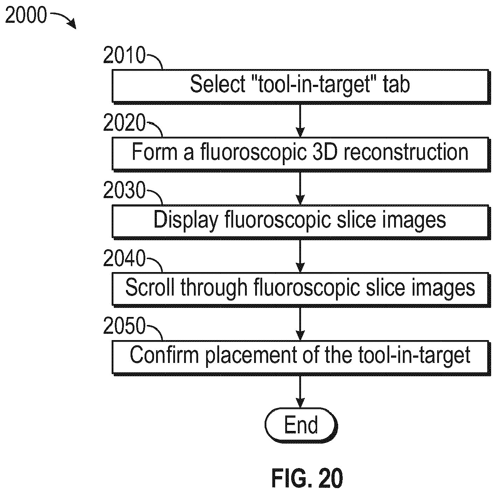

To undertake this tool-in-target confirmation, a second fluoroscopic imaging process can be undertaken. As part of this process, the user can select a "tool-in target" tab on the user interface at step 2010 of the method 2000 of Fig. 20. This selection can initiate the fluoroscopic imaging device 124. At step 2010, the user may also be directed to perform a fluoroscopic sweep similar to that described previously. The images collected during the fluoroscopic sweep can be processed to form a fluoroscopic 3D reconstruction step 2020.

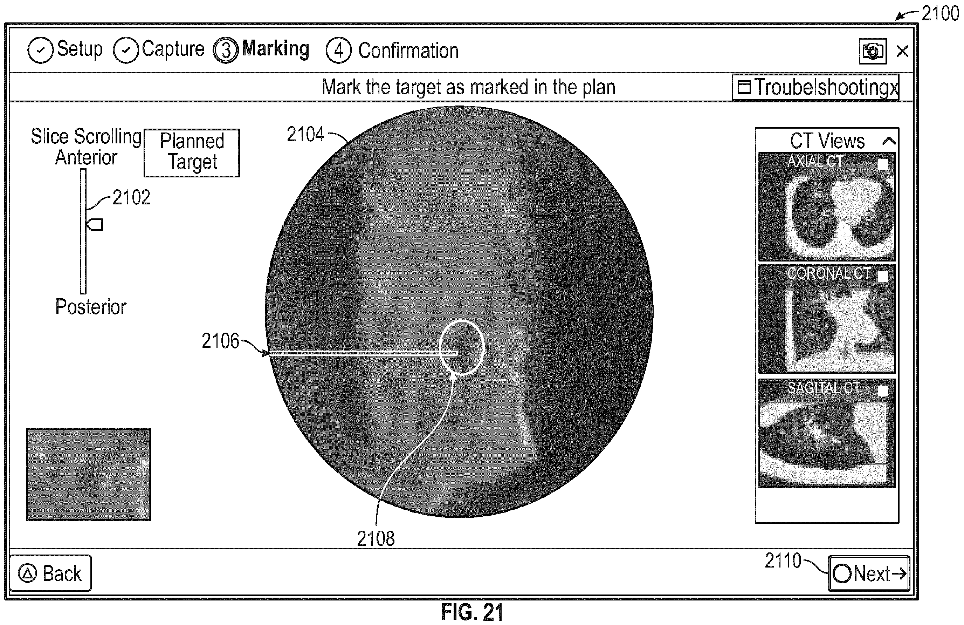

A slice of the 3D reconstruction is generated from the fluoroscopic 3D reconstruction and output as screenshot 2100 as depicted in Fig. 21 in step 210. The screen shot 2100 is similar to that of Fig. 18. The user is able to use the scroll bar 2102 to scroll through the slices of the 3D reconstruction 2104. The biopsy tools are typically made of metal and other materials which resolve quite well in fluoroscopic images. As a result, the user can scroll through the slices of the 3D reconstruction 2104 all along the sweep to ensure that the biopsy tool is in the target indicated by marker 2108 at step 2040.

As an alternative to step 2040 where the user scrolls through the slices of the 3D reconstruction 504, the user may be requested to mark the position of the catheter 102 similar to the step described in step 1760 (Fig. 78) in a 2D fluoroscopic image acquired by the fluoroscopic imaging device 124 as part of its fluoroscopic sweep from step 2110. This step may also be performed automatically via image processing techniques by computing device 122. After receiving the marked position of the catheter 102, the 3D coordinates of the marked position can be determined by the computing device 122. Accordingly, the computing device 122 can identify a slice of the 3D reconstruction 2104 that best displays the catheter 102. Additionally, or alternatively, this marking of the position of the catheter 102 in the 2D fluoroscopic image provides an indication of the position of the catheter 102 in the 3D reconstruction 2104, which can then be presented to the user for review. Still further, the user may be asked to mark the target in the 2D fluoroscopic image and a 3D relative position of the target and the catheter 102 or biopsy tool 2106 can be calculated and displayed in the 3D reconstruction 2104, similar that that described above with reference to Fig. 17.

In addition to the above, with respect to depiction of the catheter 102 in the slice images of the 3D reconstruction 2104, image processing techniques can be employed to enhance the display of the catheter 102 or biopsy tool 2106 extending therethrough. These techniques may further be employed to remove artifacts that might be the result of the reconstruction process.

If the user is satisfied with the position of the biopsy tool 2106, the user can click the next button 5210 to confirm placement of the tool-in-target at step 2050, thereby ending the tool-in-target confirmation method 2000 of Fig. 20. Then, the user can proceed back to the 3D model and perform navigation to other targets. Additionally, if more biopsies are desired, following each movement of the catheter 102 and biopsy tool 2106, one could perform another tool-in-lesion confirmation starting again at step 2110. Though not necessarily required, the system may prompt the user to identify the location of the target in the images, though most users can perform their confirmation of the tool-in-lesion without requiring a separate indication of the target.

All the heretofore described techniques of insertion of a bronchoscope, registration, navigation to a target, local registration, removal of an LG, insertion of biopsy tools, confirmation of tools in target, etc., can be experienced by a clinician or user in a virtual reality environment by employing the headset 20 and computer 30. As noted above, upon donning the headset 20, the user may experience a virtual environment as depicted in Figs. 3-5, which accurately represents an actual bronchoscopic suite or a surgical room. That virtual environment includes all the equipment necessary to undertake a procedure as described above virtually. All the screen shots of the navigation software, the CT images, the 3D models derived therefrom, and the fluoroscopic images are displayed on a display which is viewable to the clinician in the virtual environment.

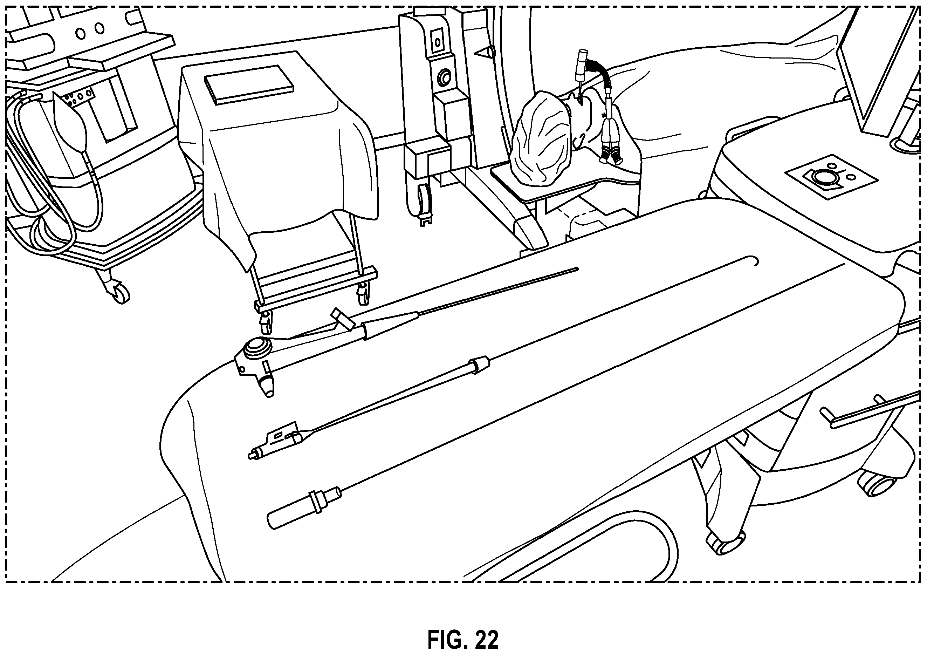

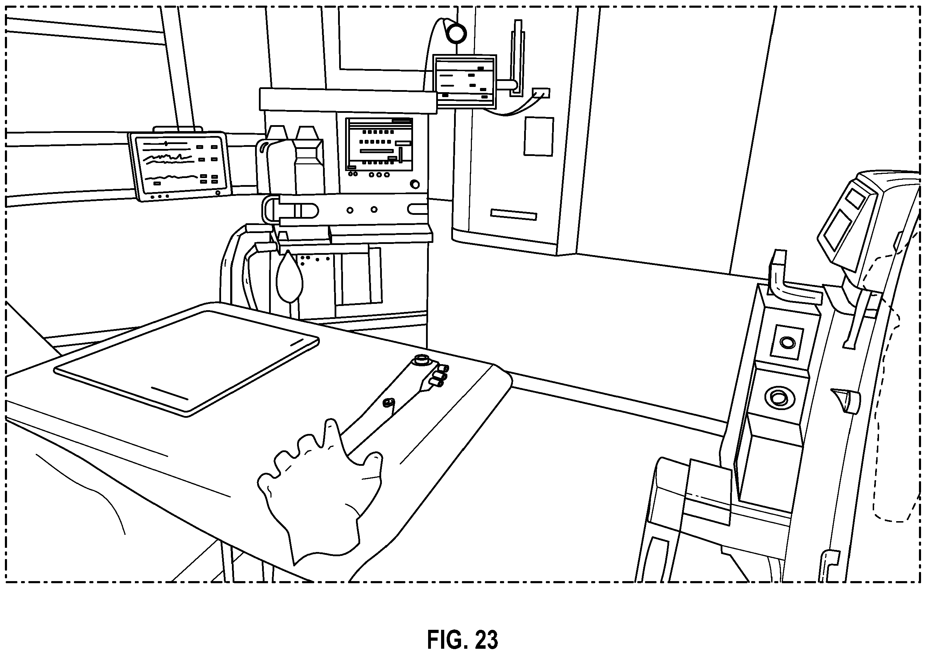

As an example, prior to initiation of a procedure, the clinician may observe that the bronchoscope 108, catheter 102 and LG 104 are set out on a tools table as depicted in Fig. 22. Upon approaching a patient, the virtual environment may present various types of guidance to the user, for example, in Fig. 23, there are displayed instructions to "pick up the bronchoscope." Using VR techniques that have been well established the physical movements of the user's hands and head are detected by the headset 20 and translated into the virtual environment allowing the user to visually experience the actions that will be required in an actual case.

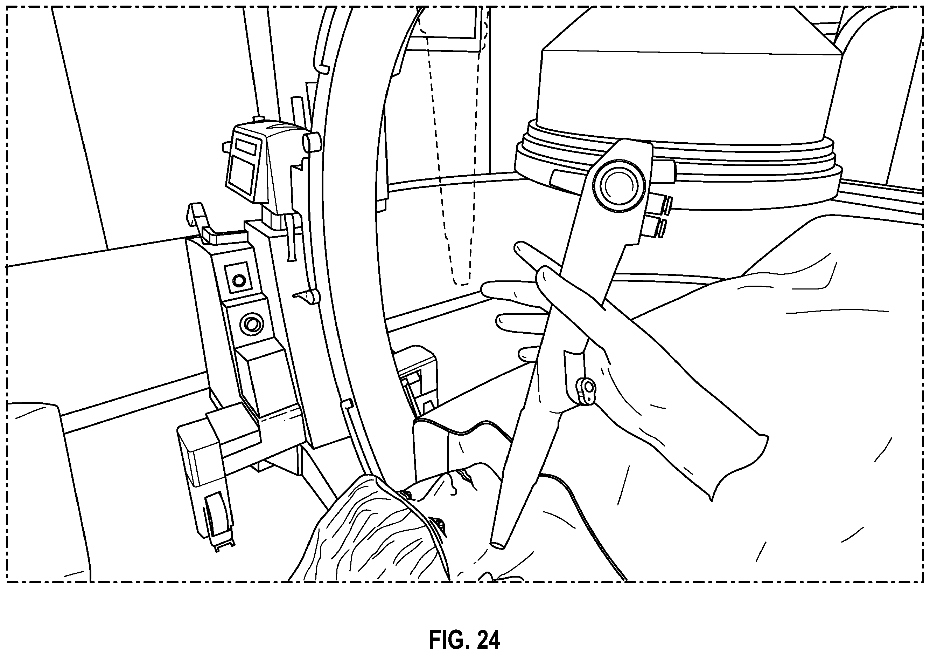

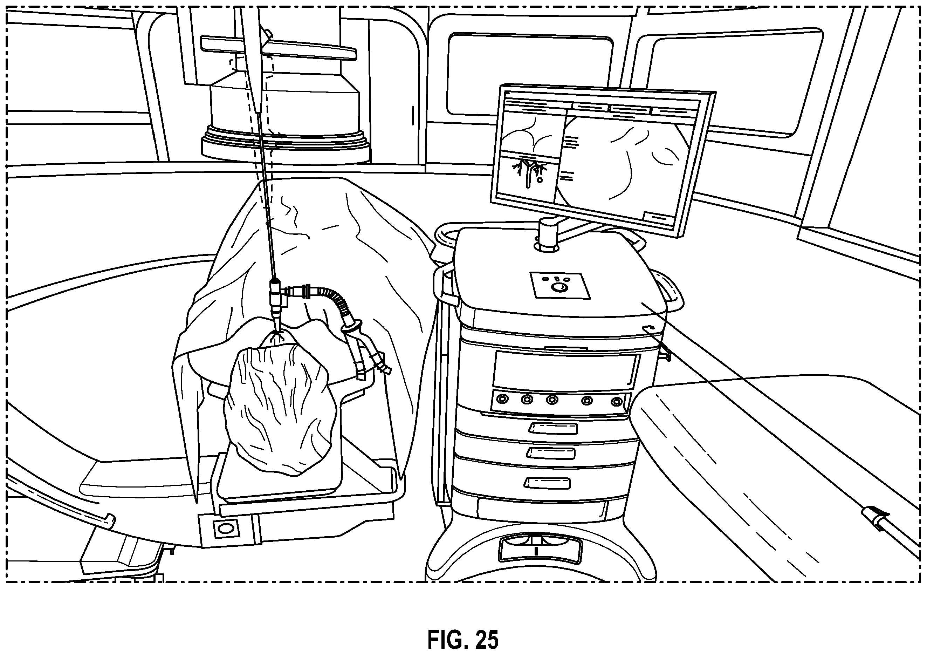

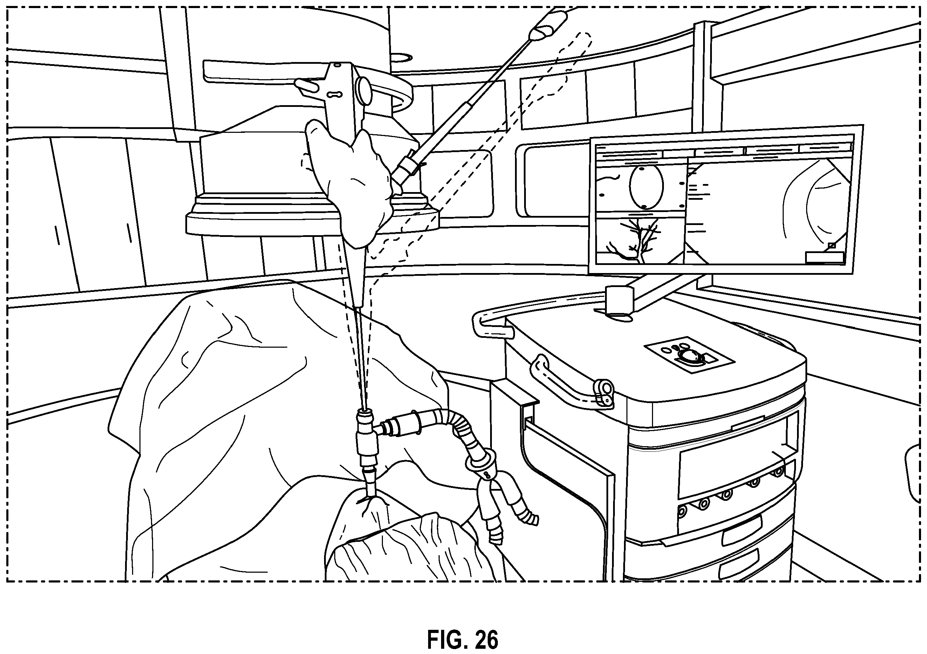

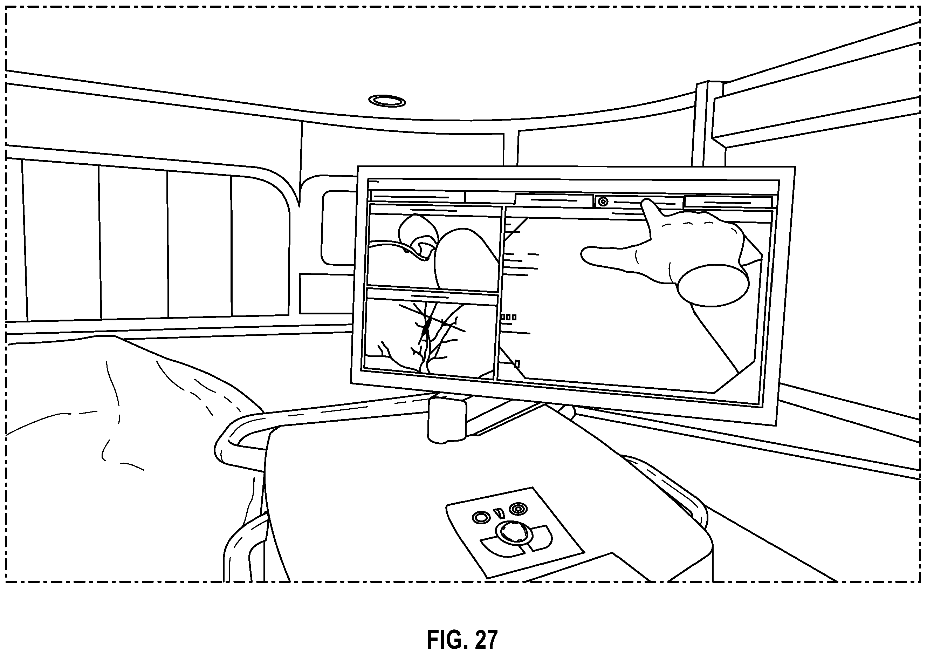

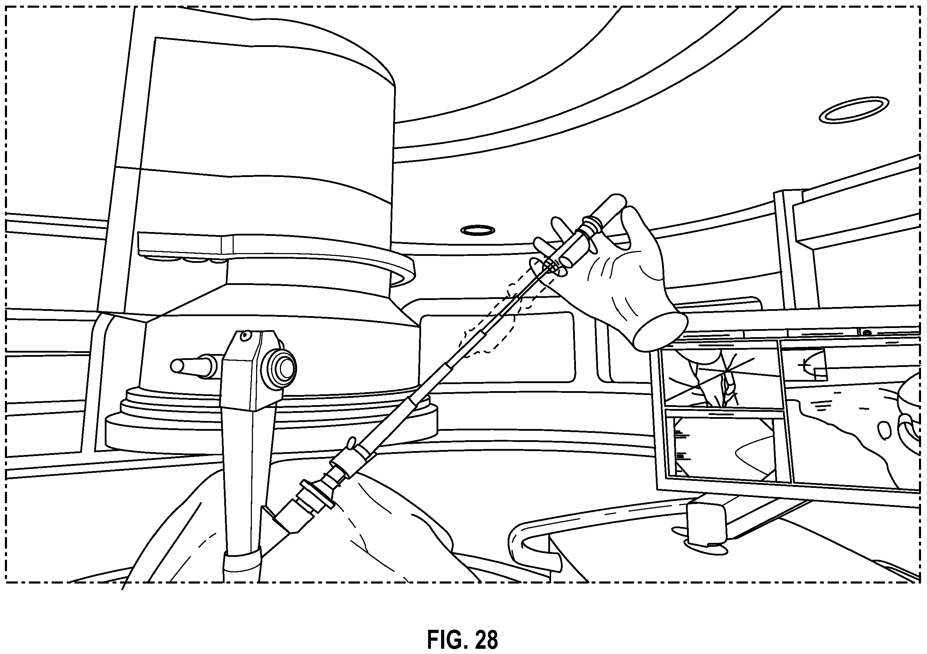

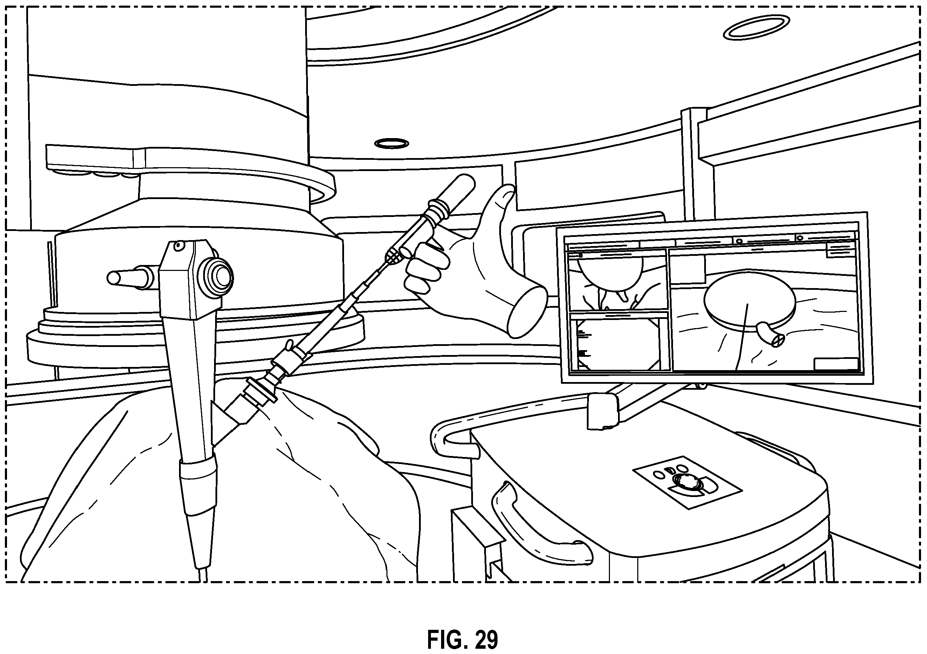

Following picking up the bronchoscope, the virtual environment may prompt the user to insert the bronchoscope into the patient as shown in Fig. 24. Additional guidance, including wireframe shapes and the like may be employed to assist the user in understanding where to place the bronchoscope relative to the patient. As depicted in Fig. 25, the representation of the bronchoscope, a wireframe for its placement and advancement, and a representation of the software application for bronchoscopic navigation are displayed. At this position, the user experiences nearly all the sensations that they would otherwise experience during an actual procedure. Following the prompts in the virtual environment and utilizing the software which is presented on the computer in the image, and which corresponds to an actual computer 122 of Fig. 2, the case can be virtually performed. At each point, either additional prompts in the virtual environment can be presented to the user as seen in Fig. 26-29, and the user interface on the computer in the virtual environment is updated. The user, as depicted in Fig. 27 can interact with the software running on the computer in the virtual environment just as they might the actual software running on computer 122. And the case proceeds until proximate a target, and the user is prompted to perform the local registration using the virtual fluoroscopic imager and all the other aspects of the software until a biopsy is acquired or the target is treated, as described herein above. All the workflows in Fig. 6 are enabled in the virtual environment.

The virtual environment may include a variety of virtual cases on which the user can practice. These are loaded onto computer 30 and selected by the user. Each case includes its unique underlying CT image data that is displayed as part of the user interface on the virtual computer and may include unique tools or prompts to the user. In addition, the unique case may include its own unique fluoroscopic data, if necessary, for local registration and the like.

By performing the virtual navigations, a user or clinician gains experience with the equipment and the workflows without the need of an actual patient. Further as part of that experience data can be collected regarding proficiency of the user, and an assessment can be made which helps ensure that clinicians are proficient with the system 100 before performing a live procedure. This may also be used for refresher courses, and continuing education evaluations to ensure that clinicians are current on the procedure and equipment. These virtual environments may also provide a means of rolling out new features and presenting them to clinicians in a manner in which they can not only hear and see them but actually experience them.

In another aspect of the disclosure, for each case there are certain metrics and thresholds programmed into the virtual environment that are used to assess the performance of the user. As the user experiences the case the computer 30 logs the performance of the user with respect to these thresholds and metrics. In some embodiments, immediately upon missing one of these thresholds or metrics the virtual environment displays an indication of the missed threshold and provides guidance on how to correct. Additionally or alternatively, the virtual environment may store these missed thresholds and metrics and present them to the user at the end of a virtual case as part of a debrief session to foster learning. Still further, all the prompts and guidance be stopped and the user's ability to navigate the virtual environment on their own, without any assistance or prompting can be assessed. It will be appreciated that for training purposes, the first few instances of utilization may be with full prompting from the virtual environment and as skill is developed these are reduced. Much like airplane pilots, a final assessment before use with the actual bronchoscopic suite and a live patient may involve a solo approach were the user must navigate the case entirely based on their experience and knowledge of the system.

Still further, as noted above, an attending nurse, additional clinician, or sales staff may also be in the virtual procedure to provide guidance or to gain their own experience with the system or to walk a new user through the process and allow them to gain familiarity. Additional uses of the VR system described herein will also be known to those of ordinary skill in the art.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

From the foregoing and with reference to the various figure drawings, those skilled in the art will appreciate that certain modifications can also be made to the disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of embodiments. Those skilled in the art will envision other modifications within the scope and spirit of the claims appended hereto.

The invention may be described by reference to the following numbered paragraphs:-
1. A training system for a medical procedure comprising:
   a virtual reality (VR) headset, including a processor and a computer readable recording media storing one or more applications thereon, the applications including instructions that when executed by the processor performs steps of:
   presenting a virtual environment viewable in the VR headset replicating a bronchoscopic suite including a patient, bronchoscopic tools, a patient, and a fluoroscope;
   depicting at least one representation of a user's hand in the virtual environment;
   providing instructions in the virtual environment viewable in the VR headset for performing a bronchoscopic navigation of the patient in the virtual environment;
   enabling interaction with a bronchoscopic navigation software on a computer displayed in the virtual environment;
   enabling interaction with the bronchoscopic tools via the representation of the user's hand; and
   executing a bronchoscopic navigation in the virtual environment, wherein as the bronchoscopic navigation is undertaken, a user interface on the computer displayed in the virtual environment is updated to simulate a bronchoscopic navigation on an actual patient.
2. A training system for a medical procedure comprising:
   a virtual reality (VR) headset; and
   a computer operably connected to the VR headset, the computer including a processor and a computer readable recording media storing one or more applications thereon, the applications including instructions that when executed by the processor performs steps of:
      presenting a virtual environment viewable in the VR headset replicating a bronchoscopic suite including a patient, bronchoscopic tools, a patient, and a fluoroscope;
      providing instructions in the virtual environment viewable in the VR headset for performing a bronchoscopic navigation of the patient in the virtual environment;
      enabling interaction with a bronchoscopic navigation software on a computer displayed in the virtual environment; and
      executing a bronchoscopic navigation in the virtual environment, wherein as the bronchoscopic navigation is undertaken, a user interface on the computer displayed in the virtual environment is updated to simulate a bronchoscopic navigation on an actual patient.
3. The training system of paragraph 2, wherein the user interface displays one or more navigation plans for selection by a user of the VR headset.
4. The training system of paragraph 3, wherein the computer in the virtual environment displays a user interface for performance of a registration of the navigation plan to a patient.
5. The training system of paragraph 4, wherein during registration the virtual environment presents a bronchoscope, catheter, and locatable guide for manipulation by a user in the virtual environment to perform the registration.
6. The training system of paragraph 5, wherein the virtual environment depicts at least one representation of a user's hands.
7. The training system of paragraph 6, wherein the virtual environment depicts the user's hands manipulating the bronchoscope, catheter, or locatable guide.
8. The training system of paragraph 6, wherein the virtual environment depicts the user's hands manipulating the user interface on the computer displayed in the virtual environment.
9. The training system of paragraph 6, wherein the computer in the virtual environment displays a user interface for performance of navigation of airways of a patient.
10. The training system of paragraph 6, wherein the user interface for performance of navigation includes central navigation, peripheral navigation, and target alignment.
11. The training system of paragraph 10, wherein the user interface for performance of navigation depicts an updated position of the locatable guide as the bronchoscope or catheter are manipulated by a user.
12. The training system of paragraph 1, further comprising a plurality of user interfaces for display on the computer in the virtual environment for performance of a local registration.
13. A method for simulating a medical procedure on a patient in a virtual reality environment, comprising:
   presenting in a virtual reality (VR) headset a virtual environment replicating a bronchoscopic suite including a patient, bronchoscopic tools, a patient, and a fluoroscope;
   providing instructions in the virtual environment viewable in the VR headset for performing a bronchoscopic navigation of the patient in the virtual environment;
   enabling interaction with a bronchoscopic navigation software on a computer displayed in the virtual environment; and
   executing a bronchoscopic navigation in the virtual environment, wherein as the bronchoscopic navigation is undertaken, a user interface on the computer displayed in the virtual environment is updated to simulate a bronchoscopic navigation on an actual patient.
14. The method of paragraph 13, wherein the virtual environment depicts at least one representation of a user's hands.
15. The method of paragraph 14, wherein the virtual environment depicts the representation of the user's hands manipulating the bronchoscope, catheter, or locatable guide.
16. The method of paragraph 14, wherein the virtual environment depicts the representation of the user's hands manipulating the user interface on the computer displayed in the virtual environment.
17. The method of paragraph 14, wherein the computer in the virtual environment displays a user interface for performance of navigation of airways of a patient.
18. The method of paragraph 17, wherein the user interface for performance of navigation includes central navigation, peripheral navigation, and target alignment.
19. The method of paragraph 18, wherein the user interface for performance of navigation depicts an updated position of a catheter within the patient as the catheter is manipulated by a representation of the user's hands.
20. The method of paragraph 12, further comprising a plurality of user interfaces for display on the computer in the virtual environment for performance of a local registration.

## Claims

1. A training system for a medical procedure comprising:
a virtual reality (VR) headset, including a processor and a computer readable recording media storing one or more applications thereon, the applications including instructions that when executed by the processor performs steps of:
presenting a virtual environment viewable in the VR headset replicating a bronchoscopic suite including a patient, bronchoscopic tools, a patient, and a fluoroscope;
depicting at least one representation of a user's hand in the virtual environment;
providing instructions in the virtual environment viewable in the VR headset for performing a bronchoscopic navigation of the patient in the virtual environment;
enabling interaction with a bronchoscopic navigation software on a computer displayed in the virtual environment;
enabling interaction with the bronchoscopic tools via the representation of the user's hand; and
executing a bronchoscopic navigation in the virtual environment, wherein as the bronchoscopic navigation is undertaken, a user interface on the computer displayed in the virtual environment is updated to simulate a bronchoscopic navigation on an actual patient.

2. A training system for a medical procedure comprising:
a virtual reality (VR) headset; and
a computer operably connected to the VR headset, the computer including a processor and a computer readable recording media storing one or more applications thereon, the applications including instructions that when executed by the processor performs steps of:
presenting a virtual environment viewable in the VR headset replicating a bronchoscopic suite including a patient, bronchoscopic tools, a patient, and a fluoroscope;
providing instructions in the virtual environment viewable in the VR headset for performing a bronchoscopic navigation of the patient in the virtual environment;
enabling interaction with a bronchoscopic navigation software on a computer displayed in the virtual environment; and
executing a bronchoscopic navigation in the virtual environment, wherein as the bronchoscopic navigation is undertaken, a user interface on the computer displayed in the virtual environment is updated to simulate a bronchoscopic navigation on an actual patient.

3. The training system of claim 2, wherein the user interface displays one or more navigation plans for selection by a user of the VR headset.

4. The training system of claim 3, wherein the computer in the virtual environment displays a user interface for performance of a registration of the navigation plan to a patient.

5. The training system of claim 4, wherein during registration the virtual environment presents a bronchoscope, catheter, and locatable guide for manipulation by a user in the virtual environment to perform the registration.

6. The training system of claim 5, wherein the virtual environment depicts at least one representation of a user's hands.

7. The training system of claim 6, wherein the virtual environment depicts the user's hands manipulating the bronchoscope, catheter, or locatable guide; and/or wherein the virtual environment depicts the user's hands manipulating the user interface on the computer displayed in the virtual environment.

8. The training system of claim 6 or claim 7, wherein the computer in the virtual environment displays a user interface for performance of navigation of airways of a patient.

9. The training system of any of claims 6 to 8, wherein the user interface for performance of navigation includes central navigation, peripheral navigation, and target alignment; preferably wherein the user interface for performance of navigation depicts an updated position of the locatable guide as the bronchoscope or catheter are manipulated by a user.

10. The training system of any preceding claim, further comprising a plurality of user interfaces for display on the computer in the virtual environment for performance of a local registration.

11. A method for simulating a medical procedure on a patient in a virtual reality environment, comprising:
presenting in a virtual reality (VR) headset a virtual environment replicating a bronchoscopic suite including a patient, bronchoscopic tools, a patient, and a fluoroscope;
providing instructions in the virtual environment viewable in the VR headset for performing a bronchoscopic navigation of the patient in the virtual environment;
enabling interaction with a bronchoscopic navigation software on a computer displayed in the virtual environment; and
executing a bronchoscopic navigation in the virtual environment, wherein as the bronchoscopic navigation is undertaken, a user interface on the computer displayed in the virtual environment is updated to simulate a bronchoscopic navigation on an actual patient.

12. The method of claim 11, wherein the virtual environment depicts at least one representation of a user's hands; preferably wherein the virtual environment depicts the representation of the user's hands manipulating the bronchoscope, catheter, or locatable guide.

13. The method of claim 11 or claim 12, wherein the virtual environment depicts the representation of the user's hands manipulating the user interface on the computer displayed in the virtual environment; preferably wherein the computer in the virtual environment displays a user interface for performance of navigation of airways of a patient.

14. The method of claim 13, wherein the user interface for performance of navigation includes central navigation, peripheral navigation, and target alignment; preferably wherein the user interface for performance of navigation depicts an updated position of a catheter within the patient as the catheter is manipulated by a representation of the user's hands.

15. The method of any of claims 11 to 14, further comprising a plurality of user interfaces for display on the computer in the virtual environment for performance of a local registration.
